# EUROPEAN PATENT SPECIFICATION

(11) **EP 4 693 317 B1**
(45) Date of publication and mention of the grant of the patent: **14.10.2026**
(21) Application number: 24779436.5
(22) Date of filing: 12.03.2024
(51) Int. Cl.: G06T 7/00, G06V 10/82, G16H 20/10, G16H 30/20, G16H 30/40, G16H 40/67, G16H 50/20, G16H 50/70, G16H 10/40

(54) **DRUG DISCOVERY ASSISTANCE APPARATUS, METHOD FOR OPERATING DRUG DISCOVERY ASSISTANCE APPARATUS, AND PROGRAM FOR OPERATING DRUG DISCOVERY ASSISTANCE APPARATUS**
VORRICHTUNG ZUR UNTERSTÜTZUNG DER ARZNEIMITTELENTDECKUNG, VERFAHREN ZUM BETRIEB DER VORRICHTUNG ZUR UNTERSTÜTZUNG DER ARZNEIMITTELENTDECKUNG UND PROGRAMM ZUM BETRIEB DER VORRICHTUNG ZUR UNTERSTÜTZUNG DER ARZNEIMITTELENTDECKUNG
APPAREIL D'AIDE À LA DÉCOUVERTE DE MÉDICAMENT, PROCÉDÉ POUR FAIRE FONCTIONNER UN APPAREIL D'AIDE À LA DÉCOUVERTE DE MÉDICAMENT, ET PROGRAMME POUR FAIRE FONCTIONNER UN APPAREIL D'AIDE À LA DÉCOUVERTE DE MÉDICAMENT

(30) Priority: 31.03.2023 JP 2023057996
(43) Date of publication of application: 11.02.2026
(73) Proprietor: FUJIFILM Corporation, Tokyo 106-8620 (JP)
(72) Inventor: TOMINAGA, Shunsuke, Ashigarakami-gun, Kanagawa 258-8577 (JP)
(74) Representative: HGF
(86) International application number: PCT/JP2024/009659
(87) International publication number: WO 2024/203307

(56) References cited:
- CA-A1- 3 225 860
- JP-A- 2015 533 210
- JP-A- 2022 112 407
- JP-A- 2022 147 007
- AZIZ MARYAM ET AL: "On Multi-Armed Bandit Designs for Dose-Finding Trials", 7 April 2020 (2020-04-07), pages 1 - 36, XP093346294, Retrieved from the Internet <URL:https://arxiv.org/pdf/1903.07082> [retrieved on 20251211]
- KUKLYTE JOGILE ET AL: "Evaluation of the Use of Single- and Multi-Magnification Convolutional Neural Networks for the Determination and Quantitation of Lesions in Nonclinical Pathology Studies", TOXICOLOGIC PATHOLOGY., vol. 49, no. 4, 23 February 2021 (2021-02-23), US, pages 815 - 842, XP093128676, ISSN: 0192-6233, DOI: 10.1177/0192623320986423

## Description

### BACKGROUND OF THE INVENTION

### 1. Field of the Invention

The technology of the present disclosure relates to a drug discovery support device, a method for operating a drug discovery support device, and a program for operating a drug discovery support device.

### 2. Description of the Related Art

In the field of drug discovery, a test is conducted in which a candidate substance for a drug is administered to a subject such as a rat, and the efficacy and toxicity of the candidate substance are evaluated. In such an evaluation test, a specimen image of a tissue specimen of an organ (a brain specimen, a liver specimen, a heart specimen, or the like) collected by autopsying a subject is used. To be more specific, a morphological abnormality occurred in a tissue specimen in a specimen image is detected. Hitherto, a user such as a pathologist observes a specimen image to detect a portion where a morphological abnormality is estimated to have occurred (hereinafter referred to as an estimated morphological abnormality portion). With the recent progress in an image analysis technology, a technology for automatically detecting an estimated morphological abnormality portion without troubling a user has been developed.

Although a technology for automatically detecting an estimated morphological abnormality portion is used, it is a user such as a pathologist that makes a final determination as to whether a morphological abnormality has actually occurred. The number of specimen images handled in one evaluation test is, for example, several thousand. Thus, the burden on the user is still considerable.

Hitherto, the technologies described in, for example, JP2009-077800A and WO2018/008195A have been proposed as a method for efficiently analyzing an enormous number of images. JP2009-077800A describes a technology for handling multiple images captured in time series, such as multiple images captured by a capsule endoscope. In JP2009-077800A, detection of an abnormal portion is performed for each of partial regions of a first image among multiple images captured in time series. Based on a detection result of an abnormal portion in the first image, the order of performing detection of an abnormal portion in partial regions of a second image that is to be analyzed after the first image is set. To be specific, under the assumption that an abnormal portion appears in substantially the same region in the images captured in time series, a partial region where an abnormal portion has been detected in the first image and its surrounding partial regions are set to be higher in order than the other partial regions.

WO2018/008195A describes a technology for handling multiple images captured by a capsule endoscope. In WO2018/008195A, the order of performing image processing is set, based on the number of images of interest, for multiple image groups obtained by imaging the inside of the bodies of multiple subjects by using a capsule endoscope. To be specific, an image group including a relatively large number of images of interest is set to be higher in order than an image group including a relatively small number of images of interest. In addition, paragraph [0141] of WO2018/008195A describes that the order of image processing is set, based on the number of images of interest, for image groups obtained from a single subject and grouped for each of organs such as the stomach, the small intestine, and the large intestine. An image of interest is, for example, an image having many red components, an image where a lesion portion has been detected, an image having a feature quantity within a predetermined range, an image captured by a user, or the like. CA3225860A1 discloses a pathology slide prioritization method to support diagnosis. Aziz Maryam ET AL: "On Multi-Armed Bandit Designs for Dose-Finding Trials" discloses an adaptive experiment design that updates the priority selection information of each dose based on safety discovery made with previous samples. Kuklyte Jogile ET AL: "Evaluation of the Use of Single- and Multi-Magnification Convolutional Neural Networks for the Determination and Quantitation of Lesions in Nonclinical Pathology Studies" discloses automated anomaly detection in histopathology slides for multiple organs in the context of preclinical toxicity studies.

### SUMMARY OF THE INVENTION

In JP2009-077800A, the order is set based on the assumption that an abnormal portion appears in substantially the same region in the images captured in time series. Thus, it is impossible to apply this technology to a specimen image of a tissue specimen captured at one time point such as after the end of an evaluation test. Furthermore, in JP2009-077800A, an abnormal portion can be detected early in the second image, but the process of detecting an abnormal portion is useless if there is no abnormal portion in the second image.

In WO2018/008195A, it is necessary to specify an image of interest in each of multiple image groups by calculating a red component or detecting a lesion portion in all the images constituting each of the multiple image groups. When an image captured by a user is used as an image of interest, the user takes time and effort to observe the image.

One embodiment according to the technology of the present disclosure provides a drug discovery support device, a method for operating a drug discovery support device, and a program for operating a drug discovery support device that are capable of preferentially analyzing a specimen image where a morphological abnormality is estimated to have occurred in a tissue specimen, without imposing a processing load.

A drug discovery support device according to the present disclosure includes a processor. The processor is configured to obtain multiple specimen images of a tissue specimen of multiple organs of subjects subjected to an evaluation test of a candidate substance; select, in accordance with degree-of-selection-priority information in which a degree of selection priority is set for each of the multiple organs, a target specimen image of a tissue specimen of a single organ from among the multiple specimen images; make a determination as to whether a morphological abnormality has occurred in the tissue specimen in the target specimen image; and update the degree-of-selection-priority information, based on a determination result as to whether the morphological abnormality has occurred.

Preferably, in the degree-of-selection-priority information, a probability of being selected for the target specimen image is set as the degree of selection priority for each of the multiple organs.

Preferably, the processor is configured to, when the determination result indicates that the morphological abnormality has occurred, reset the degree of selection priority of the organ of the tissue specimen in the target specimen image to be higher; and when the determination result indicates that the morphological abnormality has not occurred, keep the degree of selection priority of the organ of the tissue specimen in the target specimen image unchanged or reset the degree of selection priority of the organ of the tissue specimen in the target specimen image to be lower.

Preferably, the processor is configured to, based on the determination result, reset the degree of selection priority of the organ of the tissue specimen in the target specimen image and also reset the degree of selection priority of a relevant organ having a functional relationship with the organ of the tissue specimen in the target specimen image.

Preferably, the processor is configured to receive from a user an input of a final determination result as to whether the morphological abnormality has actually occurred; and update the degree-of-selection-priority information, based on the final determination result in addition to the determination result.

Preferably, the processor is configured to, when the determination result indicates that the morphological abnormality has not occurred but the final determination result indicates that the morphological abnormality has occurred, reset the degree of selection priority of the organ of the tissue specimen in the target specimen image to be higher; and when the determination result indicates that the morphological abnormality has occurred but the final determination result indicates that the morphological abnormality has not occurred, keep the degree of selection priority of the organ of the tissue specimen in the target specimen image unchanged or reset the degree of selection priority of the organ of the tissue specimen in the target specimen image to be lower.

Preferably, the subjects are grouped into multiple groups, and in the degree-of-selection-priority information, the degree of selection priority is set for each of the multiple organs and for each of the multiple groups.

Preferably, the multiple groups include an administration group to which the candidate substance is administered and a control group to which the candidate substance is not administered.

Preferably, the administration group includes multiple sub-administration groups that are different from each other in a dose of the candidate substance.

Preferably, in the degree-of-selection-priority information in an initial state, a degree of selection priority based on knowledge that has already been obtained is set.

Preferably, the processor is configured to detect, from among portions of the target specimen image, one or more estimated morphological abnormality portions where the morphological abnormality is estimated to have occurred; and compare a numerical value related to the number of the one or more estimated morphological abnormality portions with a determination threshold value set in advance, and thus make the determination.

Preferably, the processor is configured to handle each of multiple patch images obtained by dividing the target specimen image as one of the portions; and compare feature quantities obtained by inputting the patch images to a machine learning model with reference feature quantities obtained by inputting reference patch images of a tissue specimen regarded to be normal to the machine learning model, and thus detect the one or more estimated morphological abnormality portions.

A method for operating a drug discovery support device according to the present disclosure includes obtaining multiple specimen images of a tissue specimen of multiple organs of subjects subjected to an evaluation test of a candidate substance; selecting, in accordance with degree-of-selection-priority information in which a degree of selection priority is set for each of the multiple organs, a target specimen image of a tissue specimen of a single organ from among the multiple specimen images; making a determination as to whether a morphological abnormality has occurred in the tissue specimen in the target specimen image; and updating the degree-of-selection-priority information, based on a determination result as to whether the morphological abnormality has occurred.

A program for operating a drug discovery support device according to the present disclosure causes a computer to execute a process including obtaining multiple specimen images of a tissue specimen of multiple organs of subjects subjected to an evaluation test of a candidate substance; selecting, in accordance with degree-of-selection-priority information in which a degree of selection priority is set for each of the multiple organs, a target specimen image of a tissue specimen of a single organ from among the multiple specimen images; making a determination as to whether a morphological abnormality has occurred in the tissue specimen in the target specimen image; and updating the degree-of-selection-priority information, based on a determination result as to whether the morphological abnormality has occurred.

According to the technology of the present disclosure, it is possible to provide a drug discovery support device, a method for operating a drug discovery support device, and a program for operating a drug discovery support device that are capable of preferentially analyzing a specimen image where a morphological abnormality is estimated to have occurred in a tissue specimen, without imposing a processing load.

### BRIEF DESCRIPTION OF THE DRAWINGS

Fig. 1 is a diagram illustrating a process of an evaluation test, specimen images, and a drug discovery support device;
Fig. 2 is a diagram illustrating an administration group and a control group;
Fig. 3 is a diagram illustrating an example of specimen images of tissue specimens of various organs;
Fig. 4 is a block diagram illustrating a computer constituting the drug discovery support device;
Fig. 5 is a block diagram illustrating processing units of a central processing unit (CPU) of the drug discovery support device;
Fig. 6 is a diagram illustrating selection probability information;
Fig. 7 is a diagram illustrating a process performed by a selection unit;
Fig. 8 is a diagram illustrating patch images obtained by dividing a specimen image;
Fig. 9 is a diagram illustrating a state in which feature quantities are extracted from patch images by a feature quantity extractor;
Fig. 10 is a diagram illustrating the structure of the feature quantity extractor;
Fig. 11 is a diagram illustrating a process in a learning phase of an autoencoder;
Fig. 12 is a diagram illustrating a past control group and the formation of learning reference patch images;
Fig. 13 is a diagram illustrating a state in which reference feature quantities are extracted from reference patch images by the feature quantity extractor;
Fig. 14 is a diagram illustrating a graph in which reference feature quantities are plotted in a feature quantity space, and detection reference information;
Fig. 15 is a diagram illustrating a distance between a position of a feature quantity and a representative position of reference feature quantities;
Fig. 16 is a diagram illustrating a process performed by a detection unit and a detection result;
Fig. 17 is a diagram illustrating a process performed by the detection unit and a detection result;
Fig. 18 is a diagram illustrating a process performed by a determination unit and determination reference information;
Fig. 19 is a diagram illustrating a process performed by the determination unit and a determination result;
Fig. 20 is a diagram illustrating a process performed by the determination unit and a determination result;
Fig. 21 is a diagram illustrating a process performed by an information update unit;
Fig. 22 is a diagram illustrating a state in which the information update unit resets a selection probability to be higher;
Fig. 23 is a diagram illustrating a process performed by the information update unit;
Fig. 24 is a diagram illustrating a state in which the information update unit resets a selection probability to be lower;
Fig. 25 is a diagram illustrating an analysis instruction screen;
Fig. 26 is a diagram illustrating an analysis result display screen;
Fig. 27 is a flowchart illustrating a procedure of a process performed by the drug discovery support device;
Fig. 28 is a flowchart illustrating the procedure of the process performed by the drug discovery support device;
Fig. 29 is a diagram illustrating another example of a process performed by the information update unit;
Fig. 30 is a diagram illustrating a second embodiment of resetting selection probabilities of relevant organs having a functional relationship with an organ of a tissue specimen in a target specimen image;
Fig. 31 is a diagram illustrating an analysis result display screen according to a third embodiment for receiving from a user an input of a final determination result as to whether a morphological abnormality has actually occurred, and an instruction receiving unit;
Fig. 32 is a diagram illustrating a process performed by an information update unit according to the third embodiment;
Fig. 33 is a diagram illustrating a process performed by the information update unit according to the third embodiment; and
Fig. 34 is a diagram illustrating a fourth embodiment of handling a specimen image obtained by imaging a specimen slide on which tissue specimens of multiple types of organs are placed.

### DESCRIPTION OF THE PREFERRED EMBODIMENTS

### First Embodiment

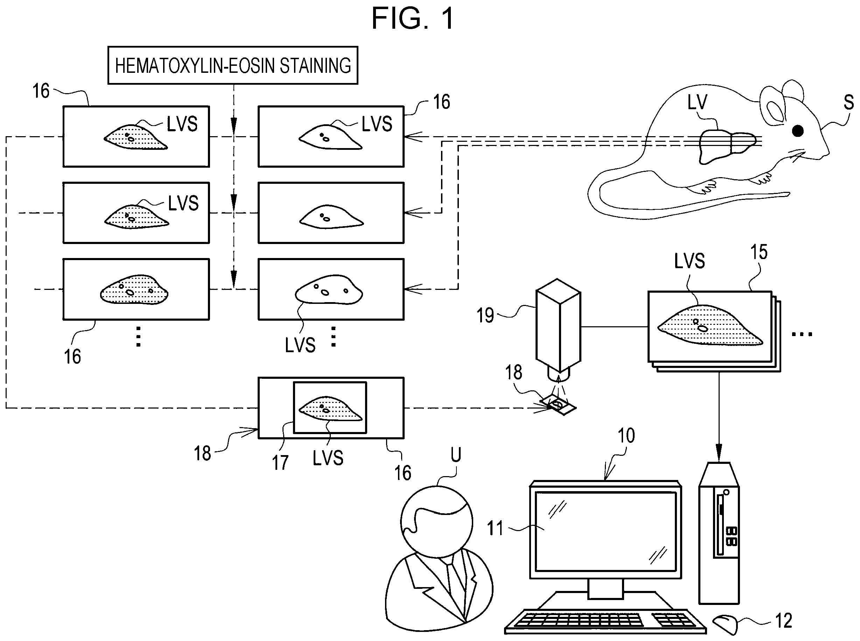

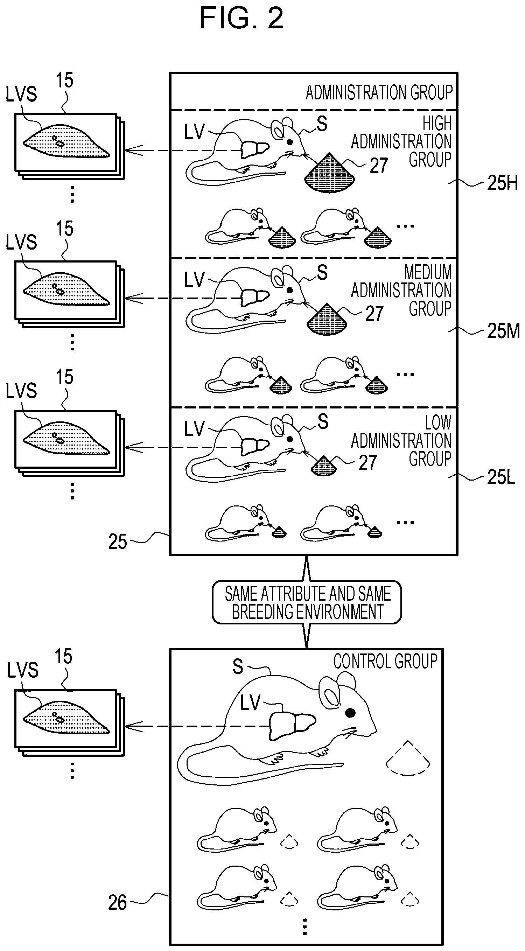

As illustrated in Fig. 1 as an example, a drug discovery support device 10 according to the present disclosure is used to evaluate the efficacy and toxicity of a candidate substance 27 (see Fig. 2) for a drug. The drug discovery support device 10 is, for example, a desktop personal computer, and includes a display 11 that displays various screens, and an input device 12 such as a keyboard, a mouse, a touch panel, and/or a microphone for voice input. The drug discovery support device 10 is installed in, for example, a drug development facility, and is operated by a user U such as a drug discovery staff member engaged in the development of a drug in the drug development facility. The drug discovery staff member includes a pathologist or the like.

The drug discovery support device 10 receives specimen images 15. The specimen images 15 are images for evaluating the efficacy and toxicity of the candidate substance 27. The specimen images 15 are generated by, for example, the following procedure. First, a subject S such as a rat prepared for evaluating the candidate substance 27 is autopsied, and multiple tissue specimens of transverse sections of an organ, a liver LV in this case, of the subject S (hereinafter referred to as liver specimens LVS) are collected. Subsequently, the collected liver specimens LVS are each applied to a glass slide 16, and then the liver specimens LVS are stained, here, with a hematoxylin-eosin stain. Subsequently, the stained liver specimens LVS are each covered with a coverslip 17 to complete specimen slides 18. Subsequently, the specimen slides 18 are each set to an image capturing device 19 such as a digital optical microscope, and the image capturing device 19 captures specimen images 15. The specimen images 15 obtained in this manner each have the whole liver specimen LVS. Thus, the specimen images 15 are called whole slide images (WSIs). The specimen images 15 are each given subject identification data (ID) for uniquely identifying the subject S, specimen image ID for uniquely identifying the specimen image 15, the date and time of capturing, and so forth. The tissue specimen is also called a tissue section. The staining may be staining with a hematoxylin stain alone, staining with a nuclear fast red stain, or the like.

As illustrated in Fig. 2 as an example, the subject S includes those belonging to an administration group 25 and those belonging to a control group 26. The administration group 25 is constituted by multiple subjects S to which the candidate substance 27 is administered. The administration group 25 is further grouped into a high administration group 25H, a medium administration group 25M, and a low administration group 25L in accordance with a dose of the candidate substance 27. As a result of grouping the administration group 25 into the high administration group 25H, the medium administration group 25M, and the low administration group 25L, it is possible to determine the influence of the dose of the candidate substance 27 on the subjects S. The high administration group 25H, the medium administration group 25M, and the low administration group 25L are an example of "sub-administration groups" according to the technology of the present disclosure. The sub-administration groups are not limited to the exemplified three groups of the high administration group 25H, the medium administration group 25M, and the low administration group 25L, and may be two groups of the high administration group 25H and the low administration group 25L, or four or more groups.

In contrast to the administration group 25, the control group 26 is constituted by multiple subjects S to which the candidate substance 27 is not administered. The number of subjects S constituting each of the high administration group 25H, the medium administration group 25M, and the low administration group 25L is the same as the number of subjects S constituting the control group 26, for example, about 5 to 10. The subjects S constituting each of the high administration group 25H, the medium administration group 25M, and the low administration group 25L and the subjects S constituting the control group 26 have the same attribute and are placed under the same breeding environment. The same attribute is, for example, the same age in weeks, the same sex, and/or the same genetic strain. The same genetic strain is, for example, the same ancestor before the fifth generation and/or the same gene sequence in a specific region. The same attribute also includes the same constituent ratio of ages in weeks, the same constituent ratio of sexes (five males and five females, for example), and/or the same constituent ratio of genetic strains. The same breeding environment is, for example, the same feed to be given, the same temperature and humidity of the breeding space, and/or the same area of the breeding space. The term "same" in the same breeding environment refers to not only completely the same, but also the same in a sense including an error that is generally accepted in the technical field to which the technology of the present disclosure pertains and that does not contradict the gist of the technology of the present disclosure.

Multiple specimen images 15 are obtained from a single subject S. Thus, the number of specimen images 15 obtained from each group is equal to the number obtained by multiplying the number of specimen images 15 obtained from a single subject S by the number of subjects S. For example, when the number of specimen images 15 obtained from a single subject S is 100 and the number of subjects S constituting each group is 10, 1000 (= 100×10) specimen images 15 are obtained from each group.

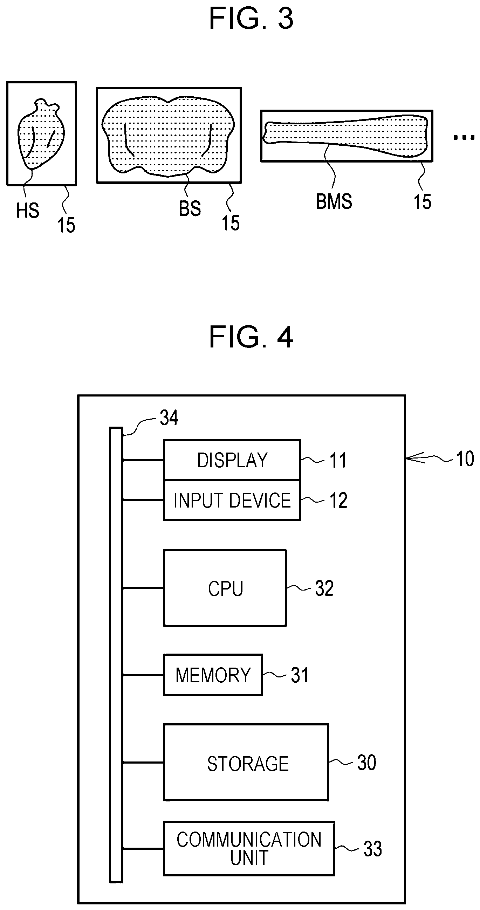

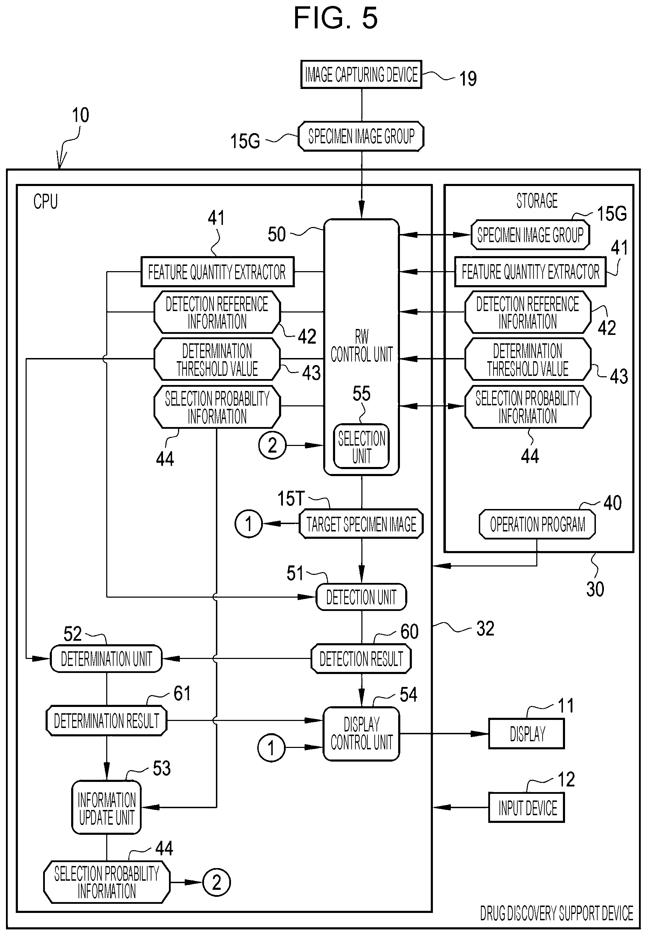

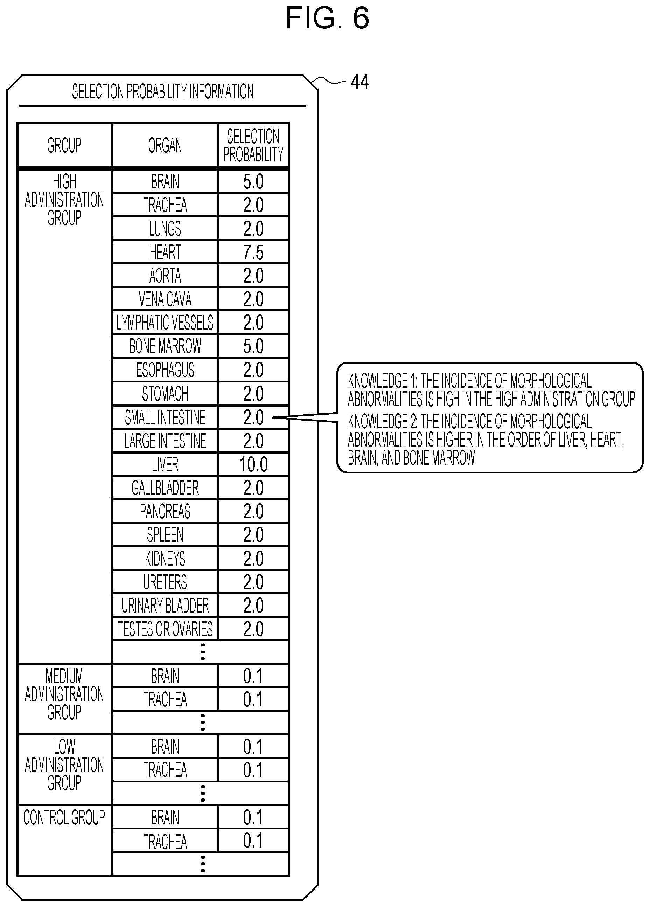

As illustrated in Fig. 3 as an example, the specimen images 15 are captured for tissue specimens of various organs of the subject S in addition to the liver specimen LVS illustrated in Fig. 1. Fig. 3 illustrates the specimen images 15 of a heart specimen HS, a brain specimen BS, and a bone marrow specimen BMS. The specimen images 15 are also captured for tissue specimens of various organs such as lungs, a stomach, a small intestine, a large intestine, a gallbladder, a pancreas, a spleen, and kidneys (see Fig. 6). There are about forty types of tissue specimens of organs captured as the specimen images 15, for example. Thus, the total number of specimen images 15 obtained from a single subject S is about several hundred. Hereinafter, a set of multiple specimen images 15 of a tissue specimen of multiple organs of multiple subjects S in each group will be referred to as a specimen image group 15G (see Fig. 5).

As illustrated in Fig. 4 as an example, the computer constituting the drug discovery support device 10 includes, in addition to the display 11 and the input device 12 described above, a storage 30, a memory 31, a central processing unit (CPU) 32, and a communication unit 33. These are connected to each other via a bus line 34.

The storage 30 is a hard disk drive built in the computer constituting the drug discovery support device 10 or connected to the computer via a cable or a network. Alternatively, the storage 30 is a disk array including multiple hard disk drives. The storage 30 stores a control program such as an operating system, various application programs, and various data associated with these programs. Alternatively, a solid-state drive may be used instead of the hard disk drive.

The memory 31 is a work memory for the CPU 32 to execute processing. The CPU 32 loads a program stored in the storage 30 into the memory 31 and executes processing in accordance with the program. Accordingly, the CPU 32 controls the individual units of the computer in a centralized manner. The CPU 32 is an example of "a processor" according to the technology of the present disclosure. The memory 31 may be built in the CPU 32. The communication unit 33 controls transmission of various pieces of information to an external device such as the image capturing device 19.

As illustrated in Fig. 5 as an example, the storage 30 of the drug discovery support device 10 stores an operation program 40. The operation program 40 is an application program for causing the computer to function as the drug discovery support device 10. That is, the operation program 40 is an example of "a program for operating a drug discovery support device" according to the technology of the present disclosure. The storage 30 also stores a feature quantity extractor 41, detection reference information 42, a determination threshold value 43, selection probability information 44, and so forth. The feature quantity extractor 41 is an example of "a machine learning model" according to the technology of the present disclosure.

Upon the operation program 40 being started, the CPU 32 of the computer constituting the drug discovery support device 10 cooperates with the memory 31 and so forth to function as a read/write (hereinafter abbreviated as RW) control unit 50, a detection unit 51, a determination unit 52, an information update unit 53, and a display control unit 54. The RW control unit 50 includes a selection unit 55.

The RW control unit 50 controls storing of various data in the storage 30 and reading of various data from the storage 30. For example, the RW control unit 50 obtains a specimen image group 15G from the image capturing device 19 and stores the obtained specimen image group 15G in the storage 30.

The selection unit 55 of the RW control unit 50 selects, in accordance with the selection probability information 44, a specimen image 15 of a tissue specimen of a single organ from the specimen image group 15G. The selection unit 55 outputs the selected specimen image 15 to the detection unit 51 and the display control unit 54. The specimen image 15 output from the selection unit 55 to the detection unit 51 and so forth is a target for determining whether a morphological abnormality has occurred in the tissue specimen. Hereinafter, the specimen image 15 serving as a target for determining whether a morphological abnormality has occurred in the tissue specimen will be referred to as a target specimen image 15T. A morphological abnormality is a lesion that is not observed in a normal tissue specimen, for example, hyperplasia, infiltration, stasis, inflammation, tumor, canceration, proliferation, bleeding, glycogen reduction, or the like.

The RW control unit 50 reads the feature quantity extractor 41 and the detection reference information 42 from the storage 30 and outputs the read feature quantity extractor 41 and detection reference information 42 to the detection unit 51. The RW control unit 50 also reads the determination threshold value 43 from the storage 30 and outputs the read determination threshold value 43 to the determination unit 52. The RW control unit 50 further reads the selection probability information 44 from the storage 30 and outputs the read selection probability information 44 to the information update unit 53.

The detection unit 51 uses the feature quantity extractor 41 and the detection reference information 42 to detect, from among portions of the target specimen image 15T, one or more estimated morphological abnormality portions where a morphological abnormality is estimated to have occurred. The detection unit 51 outputs a detection result 60 of each of the one or more estimated morphological abnormality portions to the determination unit 52 and the display control unit 54.

The determination unit 52 determines, based on the detection result 60, whether a morphological abnormality has occurred in the tissue specimen in the target specimen image 15T. The determination unit 52 outputs a determination result 61 as to whether a morphological abnormality has occurred in the tissue specimen in the target specimen image 15T to the information update unit 53 and the display control unit 54.

The information update unit 53 updates (changes) the selection probability information 44, based on the determination result 61.

The information update unit 53 outputs the updated selection probability information 44 to the RW control unit 50. The RW control unit 50 writes the updated selection probability information 44 back to the storage 30.

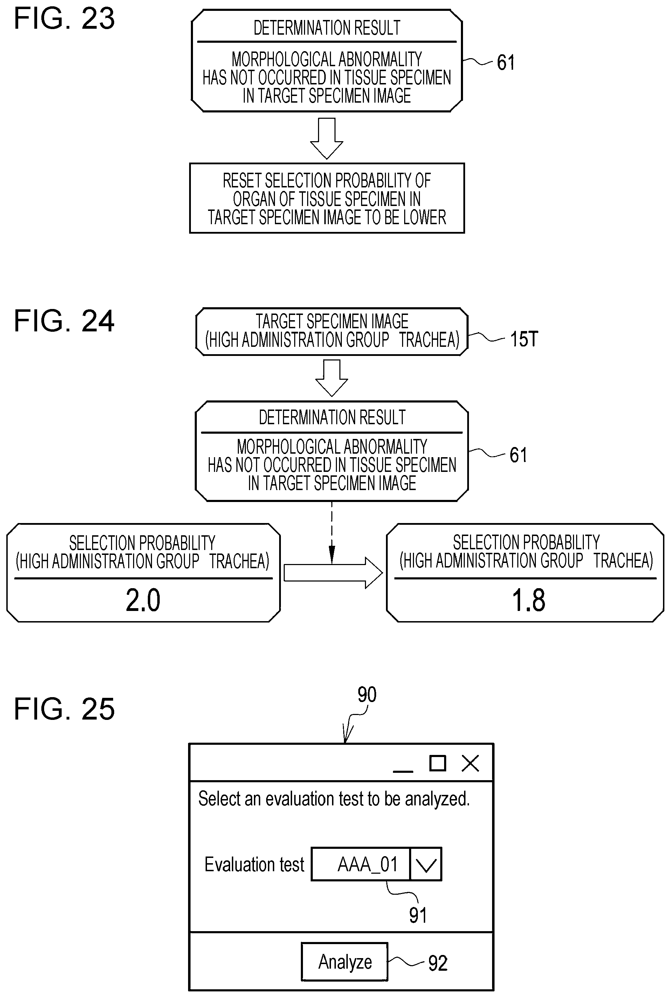

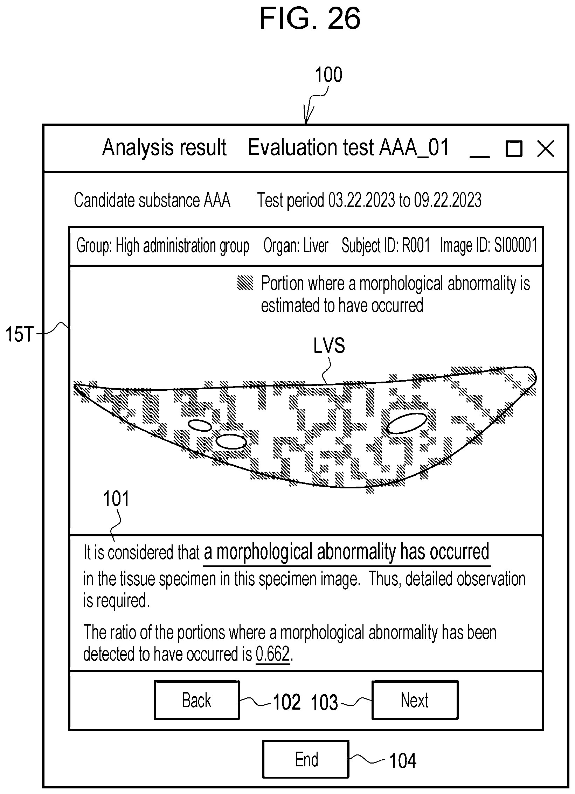

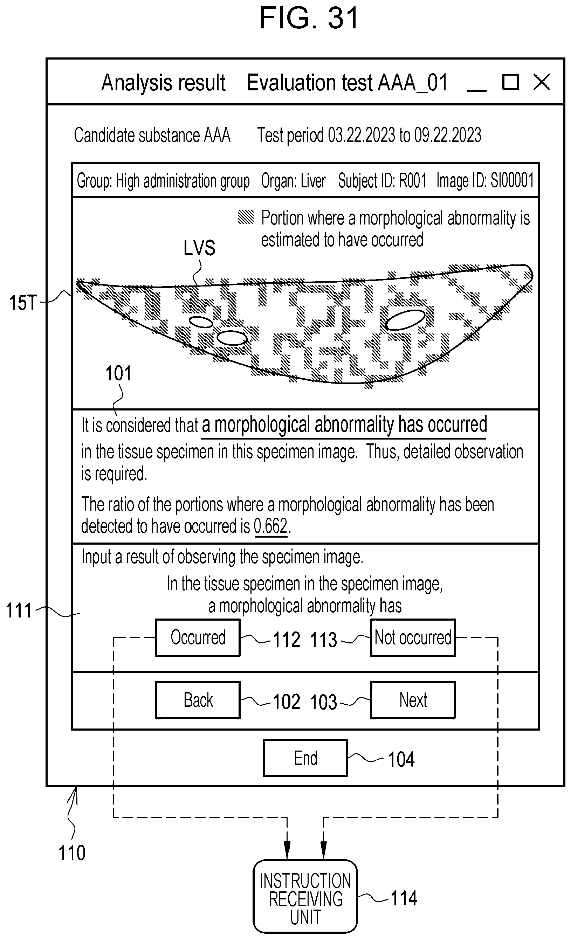

The display control unit 54 performs control to display various screens on the display 11. The various screens include an analysis instruction screen 90 (see Fig. 25) for inputting an analysis instruction, an analysis result display screen 100 (see Fig. 26), and so forth. The CPU 32 includes constructed therein an instruction receiving unit 114 (see Fig. 31) or the like that receives various operation instructions from the input device 12, in addition to the processing units 50 to 55.

As illustrated in Fig. 6 as an example, the selection probability information 44 is information in which the selection probability of each of multiple organs is registered for each of the high administration group 25H, the medium administration group 25M, the low administration group 25L, and the control group 26. The selection probability is a probability that the specimen image 15 of a tissue specimen of the organ in the group is selected as the target specimen image 15T. The selection probability is an example of "a degree of selection priority" according to the technology of the present disclosure.

The selection probability is, for example, 5.0% for the brain and 10.0% for the liver LV in the high administration group 25H. The selection probability is 0.1% for the brain and the trachea in the medium administration group 25M, the low administration group 25L, and the control group 26. Although not illustrated, 0.1% is uniformly set as the selection probability for the other organs in the medium administration group 25M, the low administration group 25L, and the control group 26. The sum of the selection probabilities of all the organs in all the groups is 100.0%. The lower limit value of the selection probability is, for example, 0.1%, and thus the selection probability is not set to 0%.

Fig. 6 illustrates the selection probability information 44 in an initial state before being updated by the information update unit 53. In the selection probability information 44 in the initial state, the selection probability is set to be higher in the high administration group 25H than in the medium administration group 25M, the low administration group 25L, and the control group 26. This is based on knowledge 1 that the incidence of morphological abnormalities is generally high in the high administration group 25H. In the selection probability information 44 in the initial state, the highest probability of 10.0% is set for the liver LV in the high administration group 25H. In addition, 7.5% is set for the heart, 5.0% is set for the brain and the bone marrow, and 2.0% is uniformly set for the other organs. This is based on knowledge 2 that the incidence of morphological abnormalities is higher in the order of the liver LV, the heart, the brain, and the bone marrow in a past evaluation test of the candidate substance 27 and a candidate substance similar thereto. As described above, in the selection probability information 44 in the initial state, the selection probabilities based on the knowledge that has already been obtained are set. The similar candidate substance is a candidate substance similar in composition to the candidate substance 27.

The knowledge may be related to a group or an organ having a high incidence of morphological abnormalities, such as the exemplified knowledge 1 and knowledge 2, or may be related to a group or an organ having a low incidence of morphological abnormalities. In the latter case, the selection probability of a group or an organ having a low incidence of morphological abnormalities is set to be relatively low.

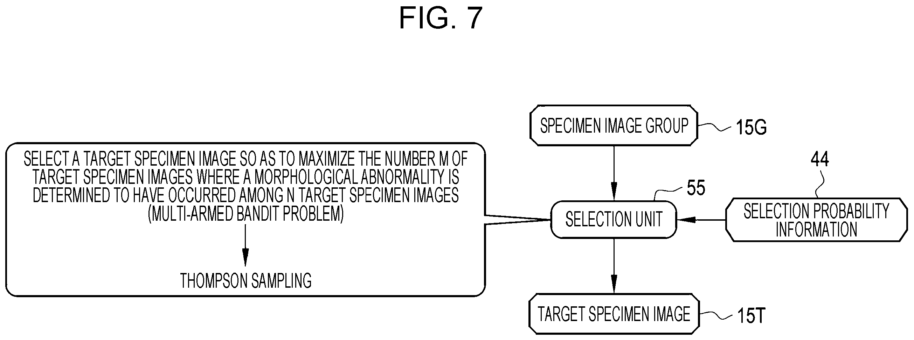

As illustrated in Fig. 7 as an example, the selection unit 55 considers a multi-armed bandit problem described below when selecting a target specimen image 15T based on the selection probability information 44. The multi-armed bandit problem is selecting a target specimen image 15T so as to maximize the number M of target specimen images 15T for which the determination unit 52 determines that a morphological abnormality has occurred in the tissue specimen among selected N target specimen images 15T. The selection unit 55 operates to clear the above-described multi-armed bandit problem by using a well-known sampling method such as Thompson sampling.

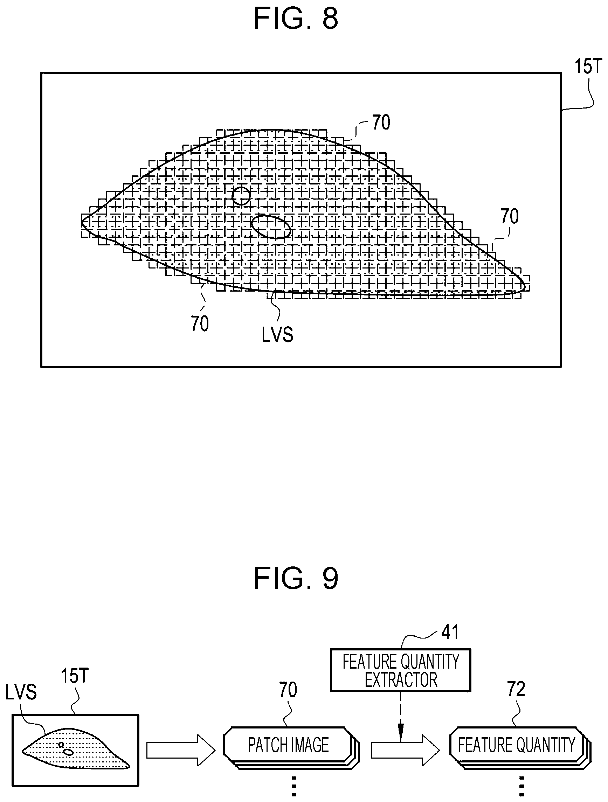

Hereinafter, a description will be given of, with reference to Fig. 8 to Fig. 15, an example of a case where a specimen image 15 of the liver specimen LVS is selected as a target specimen image 15T.

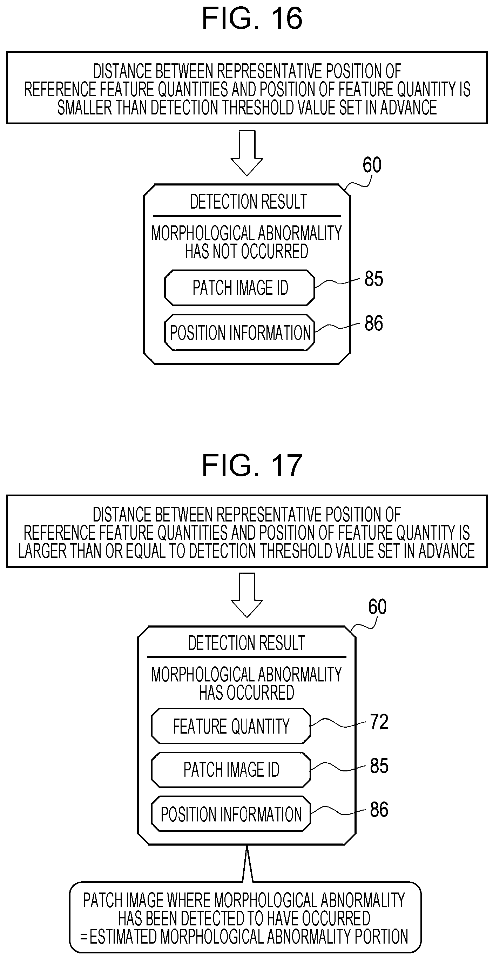

As illustrated in Fig. 8 as an example, the detection unit 51 recognizes the liver specimen LVS in the target specimen image 15T by using a known image recognition technique, and divides the recognized liver specimen LVS into multiple patch images 70. The patch images 70 each have a size that is set in advance and that can be handled by the feature quantity extractor 41. The patch images 70 each have a size that covers not only the portion of a morphological abnormality but also the peripheral region thereof. The detection unit 51 assigns patch image ID 85 (see Fig. 16 and so forth) to each patch image 70. The detection unit 51 associates the patch image ID 85 with information indicating the position of the patch image 70 in the target specimen image 15T, that is, position information 86 (see Fig. 16 and so forth) of the patch image 70. The patch images 70 are an example of "portions of a target specimen image" according to the technology of the present disclosure. In Fig. 8, adjacent patch images 70 do not have an overlapping region, but the adjacent patch images 70 may partially overlap each other.

As illustrated in Fig. 9 as an example, the detection unit 51 uses the feature quantity extractor 41 to extract a feature quantity 72 of each of the multiple patch images 70 obtained by dividing the target specimen image 15T. Thus, the number of feature quantities 72 is the same as the number of patch images 70.

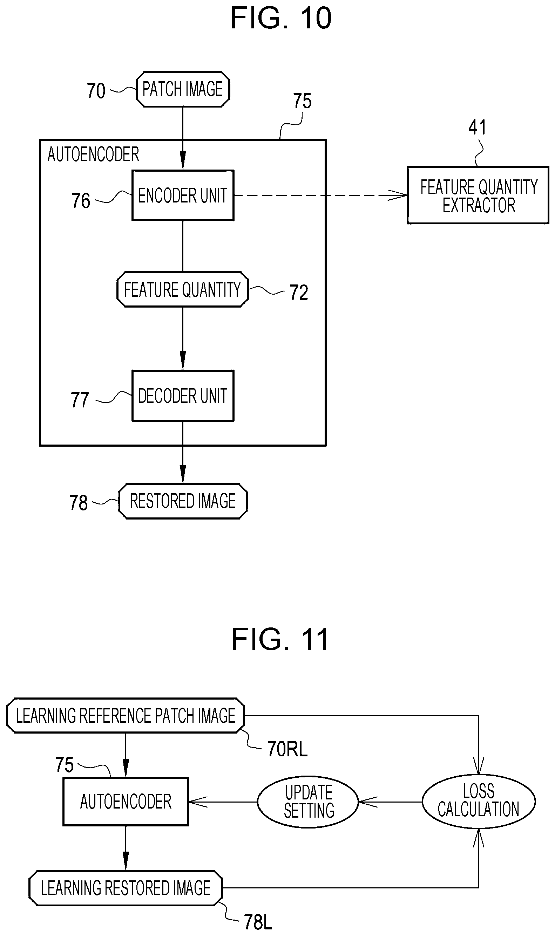

As illustrated in Fig. 10 as an example, an encoder unit 76 of an autoencoder 75 is diverted to be used as the feature quantity extractor 41. The autoencoder 75 has a decoder unit 77 in addition to the encoder unit 76. The encoder unit 76 receives a patch image 70. The encoder unit 76 converts the patch image 70 into a feature quantity 72. The encoder unit 76 transfers the feature quantity 72 to the decoder unit 77. The decoder unit 77 generates a restored image 78 of the patch image 70 from the feature quantity 72.

As is known, the encoder unit 76 has a convolutional layer that performs a convolution process using a filter, a pooling layer that performs a pooling process such as a maximum value pooling process, and so forth. The same applies to the decoder unit 77. The encoder unit 76 repeats multiple times a convolution process using the convolutional layer and a pooling process using the pooling layer on the patch image 70 input thereto, thereby extracting the feature quantity 72. The extracted feature quantity 72 represents features of the shape and texture of the liver specimen LVS in the patch image 70.

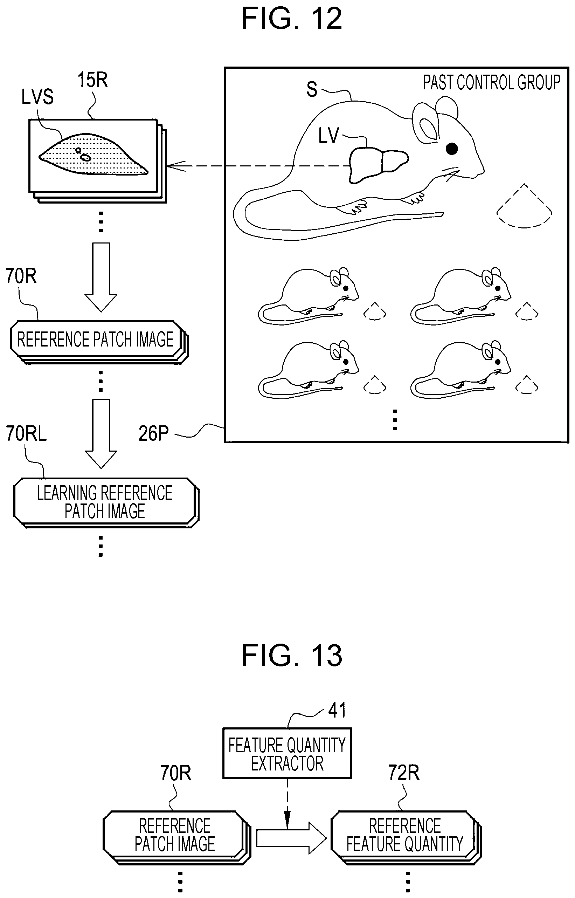

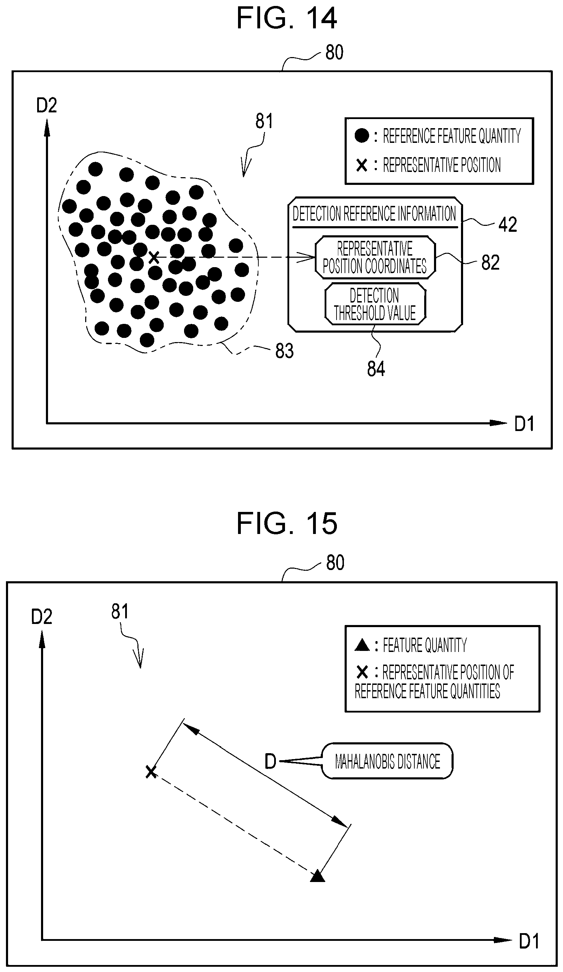

The feature quantity 72 is a set of multiple numerical values. That is, the feature quantity 72 is multidimensional data. The number of dimensions of the feature quantity 72 is, for example, 512, 1024, 2048, or the like. The feature quantity 72 and a reference feature quantity 72R (see Fig. 13) described below have the same number of dimensions, and can be compared in the same feature quantity space 81 (see Fig. 14 and so forth).

As illustrated in Fig. 11 as an example, the autoencoder 75 receives a learning reference patch image 70RL and learns in a learning phase before the encoder unit 76 is diverted to be used as the feature quantity extractor 41. The autoencoder 75 outputs a learning restored image 78L in response to receiving the learning reference patch image 70RL. Based on the learning reference patch image 70RL and the learning restored image 78L, loss calculation of the autoencoder 75 using a loss function is performed. Subsequently, update setting of various coefficients (a coefficient of a filter of the convolutional layer and so forth) of the autoencoder 75 is performed in accordance with the result of the loss calculation, and the autoencoder 75 is updated in accordance with the update setting.

In the learning phase of the autoencoder 75, the above-described series of processes including input of the learning reference patch image 70RL to the autoencoder 75, output of the learning restored image 78L from the autoencoder 75, loss calculation, update setting, and update of the autoencoder 75, is repeatedly performed while the learning reference patch image 70RL is replaced. The repetition of the above-described series of processes is terminated when the restoration accuracy from the learning reference patch image 70RL to the learning restored image 78L has reached a predetermined set level. The encoder unit 76 of the autoencoder 75 whose restoration accuracy has reached the set level is stored as the feature quantity extractor 41 in the storage 30 of the drug discovery support device 10. The learning may be terminated when the above-described series of processes has been repeated a set number of times regardless of the restoration accuracy from the learning reference patch image 70RL to the learning restored image 78L.

Such learning of the autoencoder 75 may be performed by the drug discovery support device 10 or by a device different from the drug discovery support device 10. In the latter case, the feature quantity extractor 41 is transmitted from the different device to the drug discovery support device 10, and the feature quantity extractor 41 is stored in the storage 30 by the RW control unit 50.

As illustrated in Fig. 12 as an example, learning reference patch images 70RL are supplied from multiple reference patch images 70R obtained by dividing a reference specimen image 15R. The reference specimen image 15R is an image of the liver specimen LVS of the subject S in a past control group 26P. The past control group 26P is constituted by multiple subjects S to which a candidate substance was not administered in a past evaluation test. Thus, the number of subjects S constituting the past control group 26P is significantly larger than the number of subjects S constituting the administration group 25 and the control group 26 and is, for example, about several hundred to several thousand. Like the specimen images 15, multiple reference specimen images 15R are obtained from a single subject S. Thus, the number of reference specimen images 15R obtained from the past control group 26P is equal to the number obtained by multiplying the number of reference specimen images 15R obtained from a single subject S by the number of subjects S. The liver specimen LVS of the subject S in the past control group 26P is an example of "a tissue specimen regarded to be normal" according to the technology of the present disclosure. In addition to the specimen image 15 of the liver specimen LVS of the subject S in the past control group 26P, the specimen image 15 of the liver specimen LVS determined to be normal by an expert such as a pathologist in a past administration group constituted by multiple subjects S to which a candidate substance was administered in a past evaluation test may be employed as the reference specimen image 15R.

Next, the composition of the detection reference information 42 will be described. First, as illustrated in Fig. 13 as an example, the feature quantity extractor 41 is used to extract multiple reference feature quantities 72R from multiple reference patch images 70R that are based on all of multiple reference specimen images 15R.

A graph 80 illustrated in Fig. 14 as an example is obtained by plotting the multiple reference feature quantities 72R extracted in Fig. 13 in the feature quantity space 81. The detection reference information 42 includes the coordinates of a representative position of the reference feature quantities 72R indicated by a cross mark (hereinafter referred to as representative position coordinates) 82 in the feature quantity space 81. The representative position is, for example, a center point or an average point of a distribution 83 of the reference feature quantities 72R. The detection reference information 42 also includes a detection threshold value 84. In Fig. 14, for convenience of description, the dimensions of the feature quantity space 81 are two dimensions having a D1 axis and a D2 axis, but the actual dimensions of the feature quantity space 81 are 512 dimensions or the like, as described above. Also in Fig. 15 and so forth, for convenience of description, the dimensions of the feature quantity space 81 are represented by two dimensions.

As in the learning of the autoencoder 75, the representative position coordinates 82 of the detection reference information 42 may be derived by the drug discovery support device 10 or may be derived by a device different from the drug discovery support device 10. In the latter case, the representative position coordinates 82 are transmitted from the different device to the drug discovery support device 10, and the representative position coordinates 82 are stored in the storage 30 by the RW control unit 50.

As illustrated in Fig. 15 as an example, the detection unit 51 calculates a distance D in the feature quantity space 81 between the representative position of the reference feature quantities 72R represented by the representative position coordinates 82 in the detection reference information 42 and the position of the feature quantity 72. The detection unit 51 calculates distances D for the multiple feature quantities 72 extracted for the multiple patch images 70 obtained by dividing a single target specimen image 15T. The distance D is a Mahalanobis distance. The distance D indicates the degree of deviation of the feature quantity 72 from the reference feature quantity 72R, more specifically, the degree of deviation of the liver specimen LVS in the patch image 70 of the target specimen image 15T from the liver specimen LVS regarded to be normal. That is, as the distance D increases, the liver specimen LVS in the patch image 70 deviates more from the liver specimen LVS regarded to be normal. Thus, as the distance D increases, the probability that a morphological abnormality has occurred in the liver specimen LVS in the patch image 70 increases.

As the distance D, any one of an average value, a median value, and a maximum value of the Euclidean distances between the positions of k-nearest neighbor samples of the distribution 83 of the reference feature quantities 72R and the position of the feature quantity 72 may be calculated. Alternatively, instead of the distance D, a value obtained by subtracting, from 1.0, the cosine similarity between a vector representing the representative position of the reference feature quantities 72R and a vector representing the position of the feature quantity 72 may be calculated. The cosine similarity takes a value between -1.0 and 1.0. As the value increases, the similarity of the orientations of the vectors increases. Furthermore, instead of the distance D, a likelihood function such as a negative logarithmic likelihood may be calculated as the degree of deviation.

Although not illustrated, the feature quantity extractor 41 and the detection reference information 42 are prepared for each organ. The RW control unit 50 reads the feature quantity extractor 41 and the detection reference information 42 for the organ of the tissue specimen in the target specimen image 15T from the storage 30, and outputs the feature quantity extractor 41 and the detection reference information 42 to the detection unit 51. The detection unit 51 performs the above-described process by using the feature quantity extractor 41 and the detection reference information 42 for the organ of the tissue specimen in the target specimen image 15T.

As illustrated in Fig. 16 and Fig. 17 as an example, the detection unit 51 compares the calculated distance D with the detection threshold value 84. As illustrated in Fig. 16, when the distance D is smaller than the detection threshold value 84, the detection unit 51 detects that a morphological abnormality has not occurred in the tissue specimen in the patch image 70. The detection unit 51 outputs the detection result 60 indicating that a morphological abnormality has not occurred in the tissue specimen in the patch image 70. The detection result 60 in this case includes the patch image ID 85 and the position information 86.

On the other hand, as illustrated in Fig. 17, when the distance D is larger than or equal to the detection threshold value 84, the detection unit 51 detects that a morphological abnormality has occurred in the tissue specimen in the patch image 70. The detection unit 51 outputs the detection result 60 indicating that a morphological abnormality has occurred in the tissue specimen in the patch image 70. In this case, the detection result 60 includes the feature quantity 72 in addition to the patch image ID 85 and the position information 86. The portion of the patch image 70 where a morphological abnormality has been detected to have occurred in the tissue specimen in this way corresponds to "an estimated morphological abnormality portion" according to the technology of the present disclosure. The detection threshold value 84 may be common among the high administration group 25H, the medium administration group 25M, the low administration group 25L, and the control group 26, or may be different among these groups. The detection threshold value 84 may be common among multiple organs, or may be different among the multiple organs. Furthermore, instead of using the distance D, the cosine similarity or the likelihood function described above may be compared with the detection threshold value 84 to detect whether a morphological abnormality has occurred in the tissue specimen in the patch image 70.

As illustrated in Fig. 12 and Fig. 13, the reference feature quantities 72R are feature quantities extracted from the reference patch images 70R obtained by dividing the reference specimen image 15R of the tissue specimen (the liver specimen LVS in Fig. 12 and Fig. 13) of the subject S in the past control group 26P. Because the subject S in the past control group 26P is the subject S to which a candidate substance was not administered, at least a morphological abnormality resulting from the toxicity of the candidate substance has not occurred in the tissue specimen in the reference specimen image 15R. Accordingly, the representative position of the reference feature quantities 72R is regarded as the representative position of the feature quantity of the specimen image 15 of a normal tissue specimen. Thus, the distance D between the representative position of the reference feature quantities 72R and the position of the feature quantity 72 serves as an index indicating the degree of deviation of the tissue specimen in the patch image 70 from the normal tissue specimen, as described above. Thus, as illustrated in Fig. 16, for the patch image 70 in which the distance D is smaller than the detection threshold value 84, the detection unit 51 determines that the tissue specimen in the patch image 70 does not deviate from the normal tissue specimen and detects that a morphological abnormality has not occurred. On the other hand, as illustrated in Fig. 17, for the patch image 70 in which the distance D is larger than or equal to the detection threshold value 84, the detection unit 51 determines that the tissue specimen in the patch image 70 deviates from the normal tissue specimen and detects that a morphological abnormality has occurred.

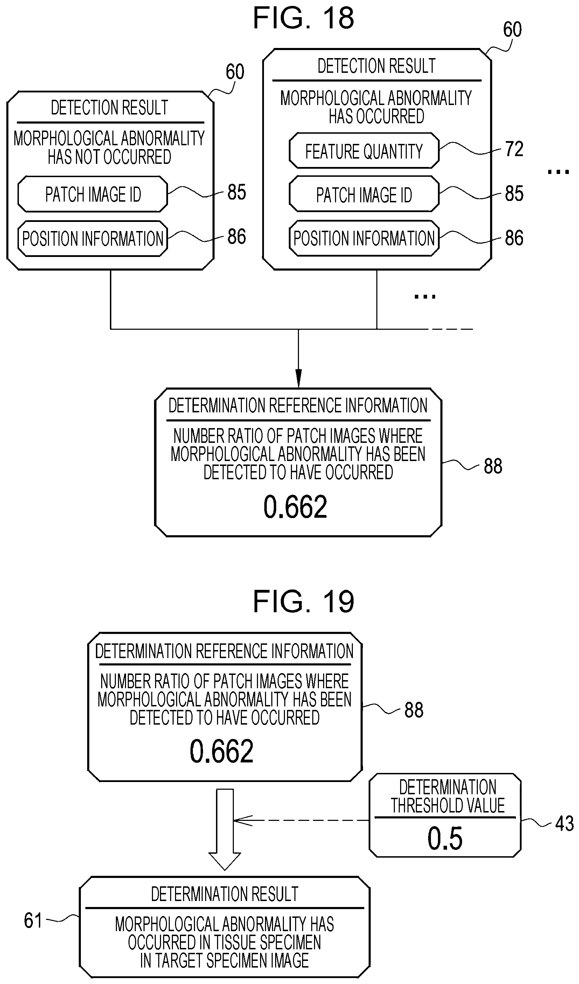

The determination unit 52 receives the detection results 60 of all the patch images 70. The determination unit 52 counts the number of detection results 60 indicating that a morphological abnormality has occurred in the tissue specimen in the patch image 70, that is, the number of estimated morphological abnormality portions. The counted number is divided by the total number of patch images 70 to calculate a number ratio of the patch images 70 where a morphological abnormality has been detected to have occurred, that is, a number ratio of estimated morphological abnormality portions. As illustrated in Fig. 18 as an example, the determination unit 52 derives the calculated number ratio as determination reference information 88. The number ratio is an example of "a numerical value related to the number of estimated morphological abnormality portions" according to the technology of the present disclosure. Instead of the number ratio, the number of estimated morphological abnormality portions may be derived as the determination reference information 88.

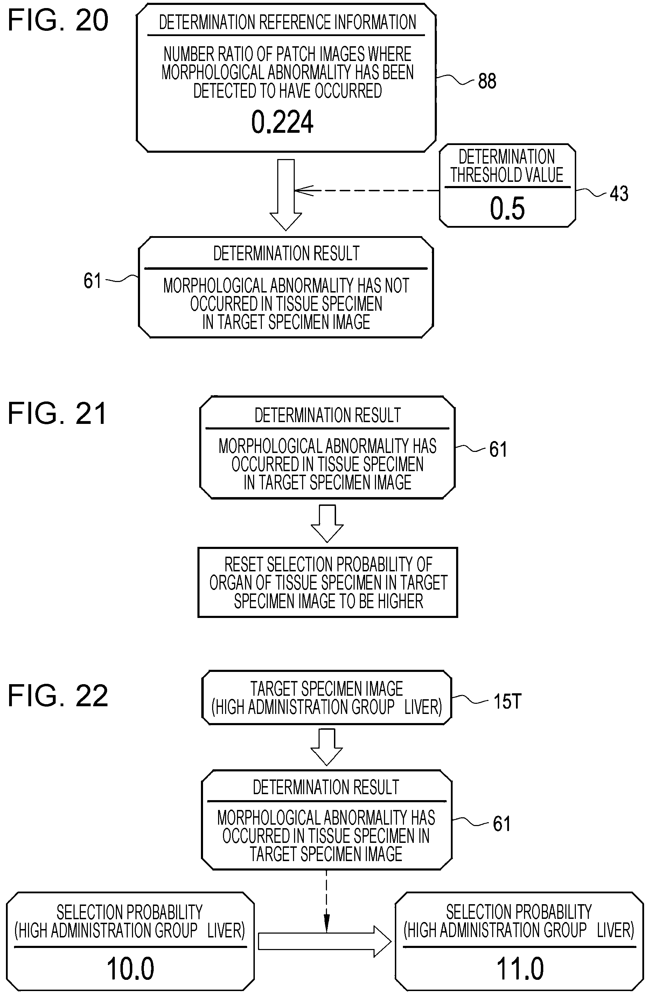

As illustrated in Fig. 19 and Fig. 20 as an example, the determination unit 52 compares the number ratio in the determination reference information 88 with the determination threshold value 43. As illustrated in Fig. 19, when the number ratio in the determination reference information 88 is larger than or equal to the determination threshold value 43, the determination unit 52 determines that a morphological abnormality has occurred in the tissue specimen in the target specimen image 15T, and outputs the determination result 61 indicating the fact.

On the other hand, as illustrated in Fig. 20, when the number ratio in the determination reference information 88 is smaller than the determination threshold value 43, the determination unit 52 determines that a morphological abnormality has not occurred in the tissue specimen in the target specimen image 15T, and outputs the determination result 61 indicating the fact. The determination threshold value 43 may be common among the high administration group 25H, the medium administration group 25M, the low administration group 25L, and the control group 26, or may be different among these groups. The determination threshold value 43 may be common among multiple organs, or may be different among the multiple organs.

As illustrated in Fig. 21 and Fig. 22 as an example, when the determination result 61 indicates that a morphological abnormality has occurred in the tissue specimen in the target specimen image 15T, the information update unit 53 resets the selection probability of the organ of the tissue specimen in the target specimen image 15T to be higher. Fig. 22 illustrates a case where the determination unit 52 has made, for the target specimen image 15T of the liver specimen LVS of the subject S in the high administration group 25H, a determination that a morphological abnormality has occurred. It is also illustrated that the selection probability of the liver LV in the high administration group 25H is reset to 11.0%, which is a 10% increase (+1.0%) from 10.0%.

On the other hand, as illustrated in Fig. 23 and Fig. 24 as an example, when the determination result 61 indicates that a morphological abnormality has not occurred in the tissue specimen in the target specimen image 15T, the information update unit 53 resets the selection probability of the organ of the tissue specimen in the target specimen image 15T to be lower. Fig. 24 illustrates a case where the determination unit 52 has made, for the target specimen image 15T of the trachea specimen of the subject S in the high administration group 25H, a determination that a morphological abnormality has not occurred. It is also illustrated that the selection probability of the trachea in the high administration group 25H is reset to 1.8%, which is a 10% decrease (-0.2%) from 2.0%. Here, the degree of increase or decrease in the selection probability (the difference between the selection probability before setting and the selection probability after setting) may be constant regardless of a group and an organ, such as 10% of the selection probability before setting as exemplified, or may be different among groups and/or organs.

As illustrated in Fig. 21 and Fig. 22, when the selection probability of a certain organ OA in a certain group GA has been reset to be higher, the information update unit 53 resets the selection probability of another organ OB in the group GA to be lower so that the total selection probability becomes 100.0%. On the other hand, as illustrated in Fig. 23 and Fig. 24, when the selection probability of a certain organ OC in a certain group GC has been reset to be lower, the information update unit 53 resets the selection probability of another organ OD in the group GC to be higher so that the total selection probability becomes 100.0%. The organ OB and the organ OD may be selected at random, or an organ having a remote relationship with the organ OA and the organ OC may be selected. An organ having a remote relationship is, for example, an organ of a different organ system such as a digestive system, a circulatory system, a urinary system, a reproductive system, or a musculoskeletal system. For example, when the organ OA is the stomach of the digestive system, the heart of the circulatory system is selected as the organ OB. The organ OB and the organ OD may be a single organ or multiple organs. The organ OB and the organ OD may be selected from a group other than the group GA and the group GC.

As illustrated in Fig. 25 as an example, upon the operation program 40 being started by the user U, for example, the display control unit 54 performs control to display the analysis instruction screen 90 on the display 11. The analysis instruction screen 90 is provided with a pull-down menu 91 for selecting an evaluation test and an analyze button 92. The user U selects a desired evaluation test in the pull-down menu 91 and then selects the analyze button 92. Accordingly, the selection of the target specimen image 15T by the selection unit 55, the detection of an estimated morphological abnormality portion by the detection unit 51, the determination of whether a morphological abnormality has occurred by the determination unit 52, and the update of the selection probability information 44 by the information update unit 53 are performed.

After the above-described processes by the individual processing units have been completed, the display control unit 54 performs control to display the analysis result display screen 100 illustrated in Fig. 26 as an example on the display 11. The target specimen image 15T is displayed on the analysis result display screen 100. On the analysis result display screen 100, the display position of the target specimen image 15T can be moved. On the analysis result display screen 100, the target specimen image 15T can be enlarged, reduced, and rotated. The display control unit 54 displays the portion of the patch image 70 detected as an estimated morphological abnormality portion by the detection unit 51 in color as indicated by hatching. The depth of the color may be increased as the difference between the distance D and the detection threshold value 84 increases.

A display region 101 is provided below the target specimen image 15T. In the display region 101, a message indicating the details of the determination result 61 made by the determination unit 52 is displayed. When the determination result 61 indicates that a morphological abnormality has occurred in the tissue specimen in the target specimen image 15T, a message prompting the user U to observe the target specimen image 15T in detail is further added as illustrated in the figure. In addition, the number ratio of estimated morphological abnormality portions calculated by the determination unit 52 is also displayed in the display region 101.

Furthermore, an image backward button 102 and an image forward button 103 are provided below the display region 101. When the user U wants to display the preceding target specimen image 15T, the user U selects the image backward button 102. On the other hand, when the user U wants to display the next target specimen image 15T, the user U selects the image forward button 103. In response to the image forward button 103 being selected, the selection of the target specimen image 15T by the selection unit 55, the detection of an estimated morphological abnormality portion by the detection unit 51, the determination of whether a morphological abnormality has occurred by the determination unit 52, and the update of the selection probability information 44 by the information update unit 53 are performed again. The display control unit 54 updates the display of the analysis result display screen 100 to the content that is based on the individual processes. The display control unit 54 hides the analysis result display screen 100 in response to an end button 104 being selected.

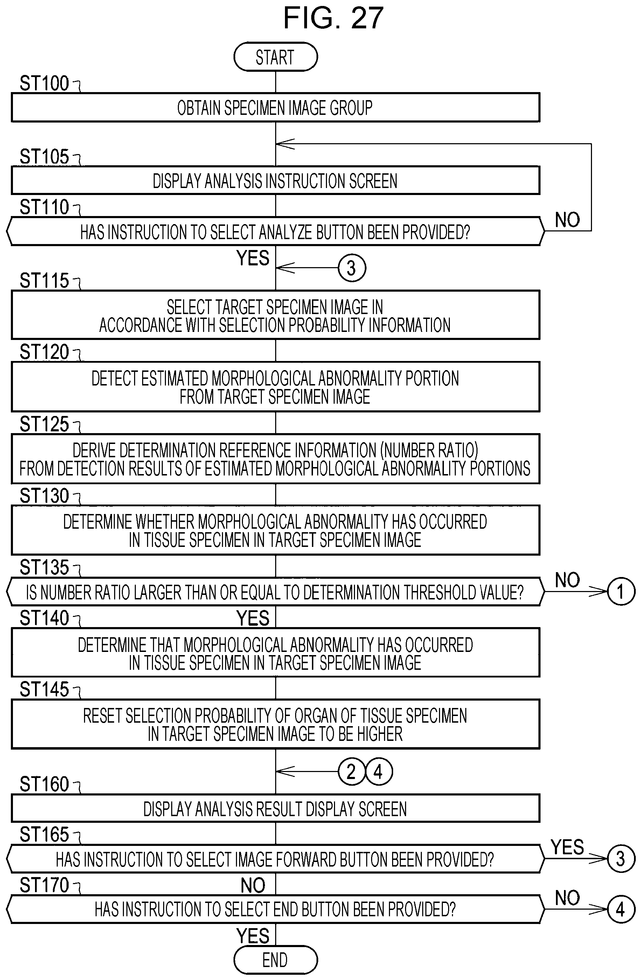

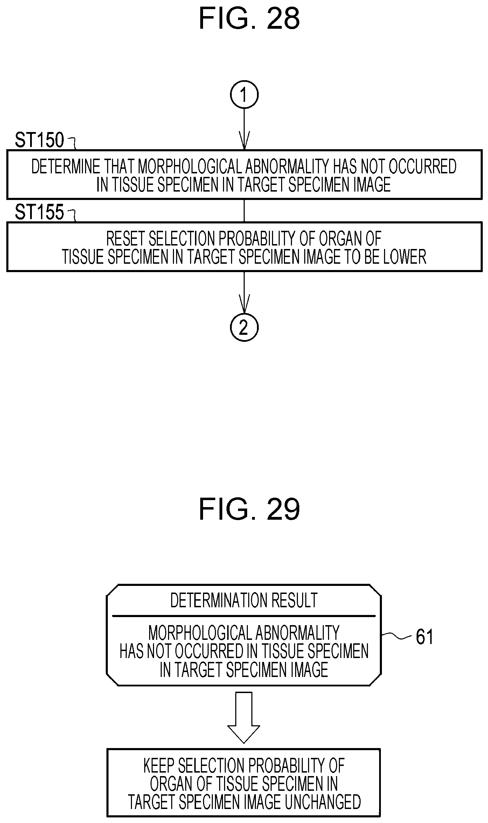

Next, the operation of the above-described configuration will be described with reference to the flowchart illustrated in Fig. 27 and Fig. 28 as an example. First, upon the operation program 40 being started in the drug discovery support device 10, the CPU 32 of the drug discovery support device 10 functions as the RW control unit 50, the detection unit 51, the determination unit 52, the information update unit 53, and the display control unit 54, as illustrated in Fig. 5. The RW control unit 50 includes the selection unit 55.

The image capturing device 19 captures specimen images 15 of a tissue specimen of multiple organs of multiple subject S in multiple groups. The multiple specimen images 15 obtained in this way, that is, a specimen image group 15G, is output from the image capturing device 19 to the drug discovery support device 10. In the drug discovery support device 10, the specimen image group 15G received from the image capturing device 19 is obtained and stored in the storage 30 by the RW control unit 50 (step ST100).

Under the control of the display control unit 54, the analysis instruction screen 90 illustrated in Fig. 25 is displayed on the display 11 (step ST105). In response to a desired evaluation test being selected in the pull-down menu 91 and the analyze button 92 being selected by the user U (YES in step ST110), the selection unit 55 selects, in accordance with the selection probability information 44, a target specimen image 15T of a tissue specimen of a single organ from among the multiple specimen images 15 constituting the specimen image group 15G, as illustrated in Fig. 7 (step ST115). The target specimen image 15T is output from the selection unit 55 to the detection unit 51 and the display control unit 54.

The RW control unit 50 reads, from the storage 30, the feature quantity extractor 41 and the detection reference information 42 for the organ of the tissue specimen in the target specimen image 15T, and outputs the read feature quantity extractor 41 and detection reference information 42 to the detection unit 51.

As illustrated in Fig. 8, the detection unit 51 divides the target specimen image 15T into multiple patch images 70. Subsequently, as illustrated in Fig. 9, the detection unit 51 extracts a feature quantity 72 from each patch image 70 by using the feature quantity extractor 41.

As illustrated in Fig. 15, the detection unit 51 calculates the distance D between the representative position of the reference feature quantities 72R and the position of the feature quantity 72. Subsequently, as illustrated in Fig. 16 and Fig. 17, the detection unit 51 compares the distance D with the detection threshold value 84 to detect whether a morphological abnormality has occurred in the tissue specimen in the patch image 70 (step ST120). The detection result 60 indicating whether a morphological abnormality has occurred in the tissue specimen in the patch image 70 is output from the detection unit 51 to the determination unit 52.

The process of extracting the feature quantity 72 from the patch image 70, the process of calculating the distance D between the representative position of the reference feature quantities 72R and the position of the feature quantity 72, and the process of detecting whether a morphological abnormality has occurred in the tissue specimen in the patch image 70 are performed on all the patch images 70 of the target specimen image 15T. After the above-described processes have been performed on all the patch images 70, the process proceeds to step ST125.

As illustrated in Fig. 18, the determination unit 52 derives the determination reference information 88 from the detection results 60 (step ST125). Subsequently, the determination unit 52 determines, based on the determination threshold value 43 and the determination reference information 88, whether a morphological abnormality has occurred in the tissue specimen in the target specimen image 15T (step ST130). To be more specific, as illustrated in Fig. 19 and Fig. 20, the number ratio of estimated morphological abnormality portions included in the determination reference information 88 is compared with the determination threshold value 43. When the number ratio is larger than or equal to the determination threshold value 43 (YES in step ST135), the process proceeds to step ST140. On the other hand, when the number ratio is smaller than the determination threshold value 43 (NO in step ST135), the process proceeds to step ST150 in Fig. 28.

In step ST140, as illustrated in Fig. 19, the determination unit 52 determines that a morphological abnormality has occurred in the tissue specimen in the target specimen image 15T. The determination result 61 is output from the determination unit 52 to the information update unit 53 and the display control unit 54. In this case, as illustrated in Fig. 21 and Fig. 22, the information update unit 53 resets the selection probability of the organ of the tissue specimen in the target specimen image 15T to be higher (step ST145). The process proceeds to step ST160.

On the other hand, in step ST150, as illustrated in Fig. 20, the determination unit 52 determines that a morphological abnormality has not occurred in the tissue specimen in the target specimen image 15T. The determination result 61 is output from the determination unit 52 to the information update unit 53 and the display control unit 54. In this case, as illustrated in Fig. 23 and Fig. 24, the information update unit 53 resets the selection probability of the organ of the tissue specimen in the target specimen image 15T to be lower (step ST155). The process proceeds to step ST160 as in the case of step ST145.

In step ST160, the analysis result display screen 100 illustrated in Fig. 26 is displayed on the display 11 under the control of the display control unit 54. Accordingly, the target specimen image 15T, the detection results 60 of estimated morphological abnormality portions, the determination result 61 of whether a morphological abnormality has occurred, and so forth are provided for the user U to view.

In response to the image forward button 103 being selected on the analysis result display screen 100 by the user U (YES in step ST165), a series of processes from step ST115 is performed again. In particular, in step ST115, the next target specimen image 15T is selected in accordance with the selection probability information 44 updated by the information update unit 53. The analysis result display screen 100 is kept displayed while the end button 104 has not been selected by the user U (NO in step ST170).

As described above, the CPU 32 of the drug discovery support device 10 includes the RW control unit 50, the selection unit 55, the determination unit 52, and the information update unit 53. The RW control unit 50 obtains multiple specimen images 15 (a specimen image group 15G) of a tissue specimen of multiple organs of subjects S subjected to an evaluation test of the candidate substance 27 for a drug. The selection unit 55 selects, in accordance with the selection probability information 44 in which a selection probability is set for each of the multiple organs, a target specimen image 15T of a tissue specimen of a single organ from among the multiple specimen images 15. The determination unit 52 determines whether a morphological abnormality has occurred in the tissue specimen in the target specimen image 15T. The information update unit 53 updates the selection probability information 44, based on the determination result 61 as to whether a morphological abnormality has occurred.

Because the selection probability information 44 is appropriately updated in accordance with the determination result 61 as to whether a morphological abnormality has occurred, the target specimen image 15T selected in accordance with the selection probability information 44 has a high probability that a morphological abnormality has occurred in the tissue specimen. Thus, the specimen image 15 where a morphological abnormality is estimated to have occurred in the tissue specimen can be preferentially analyzed without imposing a processing load. As a result, the burden on the user U who analyzes an enormous number of specimen images 15 can be reduced.

As illustrated in Fig. 6, degree-of-selection-priority information is the selection probability information 44 in which a probability of being selected for the target specimen image 15T, that is, a selection probability, is set for each of the multiple organs. Thus, the selection probability information 44 can be easily updated only by increasing or decreasing the selection probability.

The degree of selection priority is not limited to the exemplified selection probability, and may be an order. In this case, the information update unit 53 updates the degree-of-selection-priority information by making the order higher or lower based on the determination result 61.

As illustrated in Fig. 21 and Fig. 22, when the determination result 61 indicates that a morphological abnormality has occurred, the information update unit 53 resets the selection probability of the organ of the tissue specimen in the target specimen image 15T to be higher. On the other hand, as illustrated in Fig. 23 and Fig. 24, when the determination result 61 indicates that a morphological abnormality has not occurred, the information update unit 53 resets the selection probability of the organ of the tissue specimen in the target specimen image 15T to be lower. Accordingly, the selection probability of the organ in which a morphological abnormality has occurred gradually increases, and the selection probability of the organ in which a morphological abnormality has not occurred gradually decreases. Thus, it is possible to further increase the probability that the specimen image 15 where a morphological abnormality is estimated to have occurred in the tissue specimen is selected as the target specimen image 15T. As a result, the burden on the user U who analyzes an enormous number of specimen images 15 can be further reduced.

As illustrated in Fig. 2, the subjects S are grouped into multiple groups. To be more specific, the multiple groups include the administration group 25 to which the candidate substance 27 is administered and the control group 26 to which the candidate substance 27 is not administered. The administration group 25 includes the high administration group 25H, the medium administration group 25M, and the low administration group 25L that are different from each other in a dose of the candidate substance 27. As illustrated in Fig. 6, in the selection probability information 44, a selection probability is set for each of multiple organs and for each of multiple groups. Thus, the selection probability can be varied among the groups, for example, the selection probability of the high administration group 25H can be set to be higher than the selection probabilities of the other groups. Thus, it is possible to further increase the probability that the specimen image 15 where a morphological abnormality is estimated to have occurred in the tissue specimen is selected as the target specimen image 15T.

As illustrated in Fig. 6, in the selection probability information 44 in the initial state, the selection probabilities based on the knowledge that has already been obtained are set. Thus, a specimen image 15 can be selected as the target specimen image 15T in accordance with the knowledge from the beginning. Because update is started from the selection probabilities that are based on the knowledge, a specimen image 15 that matches the knowledge to some extent can be selected as the target specimen image 15T even in a case other than the initial state.

The selection probabilities in the selection probability information 44 in the initial state may have the same value in all the groups and all the organs. In this case, the first target specimen image 15T is randomly selected by the selection unit 55. The first target specimen image 15T may be selected by the user U.

As illustrated in Fig. 16 and Fig. 17, the detection unit 51 detects, from among portions of the target specimen image 15T, one or more estimated morphological abnormality portions where a morphological abnormality is estimated to have occurred in the tissue specimen. As illustrated in Fig. 19 and Fig. 20, the determination unit 52 compares a number ratio, which is a numerical value related to the number of estimated morphological abnormality portions, with the determination threshold value 43 set in advance, and thus determines whether a morphological abnormality has occurred in the tissue specimen. Thus, it is possible to accurately determine whether a morphological abnormality has occurred in the tissue specimen in the target specimen image 15T. In addition, the determination criterion is clear, and there is no possibility of erroneous determination. A machine learning model that outputs the determination result 61 in response to input of the target specimen image 15T may be used to determine whether a morphological abnormality has occurred in the tissue specimen in the target specimen image 15T.

As illustrated in Fig. 8, Fig. 9, and Fig. 15, the detection unit 51 handles each of the multiple patch images 70 obtained by dividing the target specimen image 15T as a portion. The detection unit 51 compares the feature quantities 72 obtained by inputting the patch images 70 to the feature quantity extractor 41 with the reference feature quantities 72R obtained by inputting the reference patch images 70R of the tissue specimen regarded to be normal to the feature quantity extractor 41, and thus detects the one or more estimated morphological abnormality portions. Accordingly, an estimated morphological abnormality portion can be detected easily and accurately.

### Modification

The process performed by the information update unit 53 when the determination result 61 indicates that a morphological abnormality has not occurred in the tissue specimen in the target specimen image 15T is resetting the selection probability of the organ of the tissue specimen in the target specimen image 15T to be lower, but the process is not limited thereto. As illustrated in Fig. 29 as an example, the process performed by the information update unit 53 when the determination result 61 indicates that a morphological abnormality has not occurred in the tissue specimen in the target specimen image 15T may be keeping the selection probability of the organ of the tissue specimen in the target specimen image 15T unchanged. Keeping the selection probability unchanged means that not updating the selection probability information 44. In this case, the selection unit 55 selects the next target specimen image 15T in accordance with the unchanged selection probability information 44. Accordingly, the process of updating the selection probability information 44 can be omitted, and thus the processing load can be further reduced.

An opportunity for the user U to select a target specimen image 15T may be provided between selections of a target specimen image 15T by the selection unit 55 based on the selection probability information 44. Furthermore, the following restriction may be imposed on the selection of a target specimen image 15T by the selection unit 55. That is, a target specimen image 15T is not selected from the medium administration group 25M and the low administration group 25L until a determination is made that a morphological abnormality has occurred in the tissue specimen in the target specimen image 15T of the tissue specimen of the subject S in the high administration group 25H.

### Second Embodiment

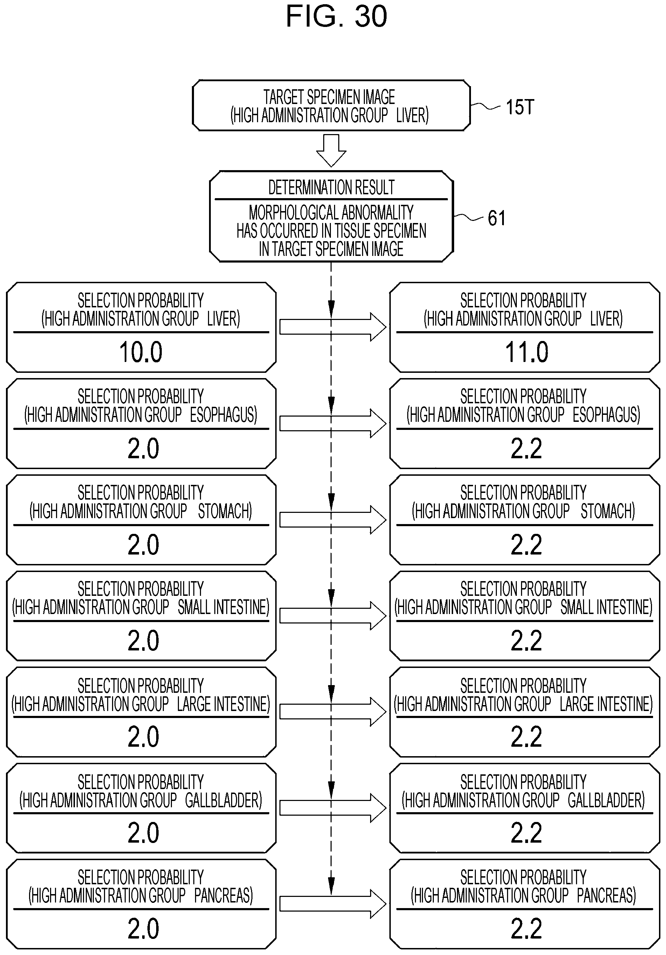

In the above-described first embodiment, only the selection probability of the organ of the tissue specimen in the target specimen image 15T is reset, but the present disclosure is not limited thereto. As in a second embodiment illustrated in Fig. 30 as an example, not only the selection probability of the organ of the tissue specimen in the target specimen image 15T but also the selection probability of a relevant organ having a functional relationship with the organ of the tissue specimen in the target specimen image 15T may be reset. Fig. 30 illustrates a case where the determination unit 52 has made, for the target specimen image 15T of the liver specimen LVS of the subject S in the high administration group 25H, a determination that a morphological abnormality has occurred. It is also illustrated that the selection probability of the liver in the high administration group 25H is reset to 11.0%, which is a 10% increase (+1.0%) from 10.0%, and that the selection probabilities of the esophagus, stomach, small intestine, large intestine, gallbladder, and pancreas in the high administration group 25H are reset to 2.2%, which is a 10% increase (+0.2%) from 2.0%.

The liver, esophagus, stomach, small intestine, large intestine, gallbladder, and pancreas are in the same organ system, that is, in the digestive system, and are relevant organs having a functional relationship. The relevant organs include the circulatory system, such as the trachea, lungs, heart, aorta, vena cava, and lymphatic vessels; the urinary system, such as the kidneys, ureters, and urinary bladder; the reproductive system, such as the testes or ovaries and genitalia; and the musculoskeletal system, such as the femurs, pectoral muscles, and bone marrow, in addition to the digestive system. Although not illustrated, when the determination unit 52 determines that a morphological abnormality has not occurred, the information update unit 53 resets the selection probabilities of the organ of the tissue specimen in the target specimen image 15T and the relevant organs thereof to be lower.

Empirically, when a morphological abnormality has occurred in a certain organ, the probability that a morphological abnormality has occurred also in a relevant organ is high. Thus, as in the second embodiment, as a result of resetting not only the selection probability of the organ of the tissue specimen in the target specimen image 15T but also the selection probabilities of the relevant organs having a functional relationship with the organ of the tissue specimen in the target specimen image 15T, it is possible to further increase the probability that the specimen image 15 in which a morphological abnormality is estimated to have occurred in the tissue specimen is selected as the target specimen image 15T.

### Third Embodiment

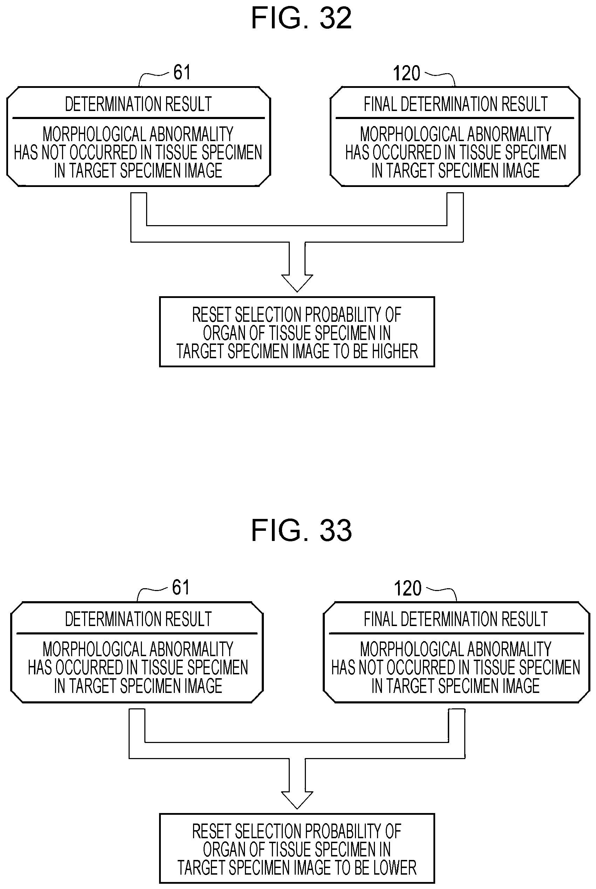

As illustrated in Fig. 31 as an example, an analysis result display screen 110 according to a third embodiment further has a display region 111 below the display region 101. In the display region 111, a message prompting the user U to input a final determination result 120 (see Fig. 32 and Fig. 33) as to whether a morphological abnormality has actually occurred in the tissue specimen in the target specimen image 15T is displayed. In addition, a first input button 112 and a second input button 113 for the user U to input the final determination result 120 are displayed in the display region 111.

In response to determining that a morphological abnormality has occurred in the tissue specimen in the target specimen image 15T as a result of observing the target specimen image 15T, the user U selects the first input button 112. On the other hand, in response to determining that a morphological abnormality has not occurred in the tissue specimen in the target specimen image 15T, the user U selects the second input button 113. An instruction to select the first input button 112 or the second input button 113, that is, the final determination result 120, is received by the instruction receiving unit 114.

As illustrated in Fig. 32 and Fig. 33 as an example, the information update unit 53 updates the selection probability information 44, based on the final determination result 120 in addition to the determination result 61. To be more specific, as illustrated in Fig. 32, when the determination result 61 indicates that a morphological abnormality has not occurred in the tissue specimen in the target specimen image 15T but the final determination result 120 indicates that a morphological abnormality has occurred in the tissue specimen in the target specimen image 15T, the information update unit 53 resets the selection probability of the organ of the tissue specimen in the target specimen image 15T to be higher. On the other hand, as illustrated in Fig. 33, when the determination result 61 indicates that a morphological abnormality has occurred in the tissue specimen in the target specimen image 15T but the final determination result 120 indicates that a morphological abnormality has not occurred in the tissue specimen in the target specimen image 15T, the information update unit 53 resets the selection probability of the organ of the tissue specimen in the target specimen image 15T to be lower. That is, the information update unit 53 updates the selection probability information 44 by emphasizing the final determination result 120 made by the user U more than the determination result 61 made by the determination unit 52. Accordingly, the final determination result 120 made by the user U can be reflected in the update of the selection probability information 44. It is possible to set a selection probability that conforms more to the determination made by the user U.

Although not illustrated, when both the determination result 61 and the final determination result 120 indicate that a morphological abnormality has occurred in the tissue specimen in the target specimen image 15T, the information update unit 53 resets the selection probability of the organ of the tissue specimen in the target specimen image 15T to be higher. Although not illustrated, when both the determination result 61 and the final determination result 120 indicate that a morphological abnormality has not occurred in the tissue specimen in the target specimen image 15T, the information update unit 53 resets the selection probability of the organ of the tissue specimen in the target specimen image 15T to be lower.

When the determination result 61 indicates that a morphological abnormality has occurred in the tissue specimen in the target specimen image 15T but the final determination result 120 indicates that a morphological abnormality has not occurred in the tissue specimen in the target specimen image 15T, the modification illustrated in Fig. 29 may be applied and the selection probability of the organ of the tissue specimen in the target specimen image 15T may be kept unchanged.

The multiple groups for which selection probabilities are set may be two groups of the administration group 25 and the control group 26. The multiple groups for which selection probabilities are set may be three groups of the high administration group 25H, the medium administration group 25M, and the low administration group 25L, and the control group 26 may be excluded. That is, the control group 26 may be excluded from the option for the target specimen image 15T.

In addition to the reference patch image 70R of a tissue specimen regarded to be normal, the patch image 70 of a tissue specimen where a morphological abnormality has occurred may also be used as the learning reference patch image 70RL. The patch image 70 of a tissue specimen where a morphological abnormality has occurred is obtained from, for example, a past administration group constituted by multiple subjects S to which a candidate substance was administered in a past evaluation test. Accordingly, the autoencoder 75 and thus the feature quantity extractor 41 is capable of learning about tissue specimens having features of more various shapes and textures. As a result, the feature quantity extractor 41 is capable of extracting a feature quantity 72 that better represents the features of the shape and texture of the tissue specimen.

The patch image 70 of a tissue specimen where a morphological abnormality has occurred is not limited to an image obtained from the subject S constituting the exemplified past administration group. A morphological abnormality may occur also in the subject S constituting the past control group 26P. Thus, it does not matter whether the subject S belongs to the past control group 26P or the past administration group as long as the patch image 70 has a tissue specimen where a morphological abnormality has occurred. Furthermore, the patch image 70 of a tissue specimen where a morphological abnormality has occurred may be an image obtained from the subject S to which various stresses are applied to intentionally cause a morphological abnormality. The patch image 70 of a tissue specimen where a morphological abnormality has occurred may be an image artificially created by processing the patch image 70 of a normal tissue specimen.

Instead of the encoder unit 76 of the autoencoder 75, an encoder unit of a convolutional neural network that outputs a class determination result in accordance with an input of the patch image 70 may be diverted to be used as the feature quantity extractor 41. The class determination result is, for example, a result of determining one type of morphological abnormality that has occurred in the tissue specimen in the patch image 70 from among multiple types such as hyperplasia, infiltration, stasis, and inflammation.

The machine learning model diverted to be used as the feature quantity extractor 41 is not limited to the exemplified autoencoder 75 and convolutional neural network. A generator of generative adversarial networks (GAN) may be diverted to be used as the feature quantity extractor 41. A machine learning model that does not have a convolutional layer, such as a vision transformer (ViT), may be diverted to be used as the feature quantity extractor 41.

Contrastive learning may be performed in which learning is performed such that the distance in a feature quantity space between feature quantities derived from the same image is short and the distance in the feature quantity space between feature quantities derived from different images is long. As the contrastive learning, for example, a learning method such as a simple framework for contrastive learning of visual representations (SimCLR) is known. Alternatively, a learning method of not using the foregoing pair of different images (also referred to as a negative sample), such as bootstrap your own latent (BYOL), may be used. The distribution of extracted feature quantities may be restricted to a distribution on a unit sphere or a distribution following a standard normal distribution.

On the analysis result display screen 100 or the analysis result display screen 110, attributes such as a group, an organ, a subject ID, and an image ID of the target specimen image 15T to be selected next may be displayed in advance. In addition, an expected time for the remaining analysis for each group and/or each organ may be displayed. The expected time can be derived from the number of specimen images 15 selected as the target specimen image 15T so far, the total number of specimen images 15 of each group and/or each organ, and an average time taken to analyze a single target specimen image 15T.

### Fourth Embodiment

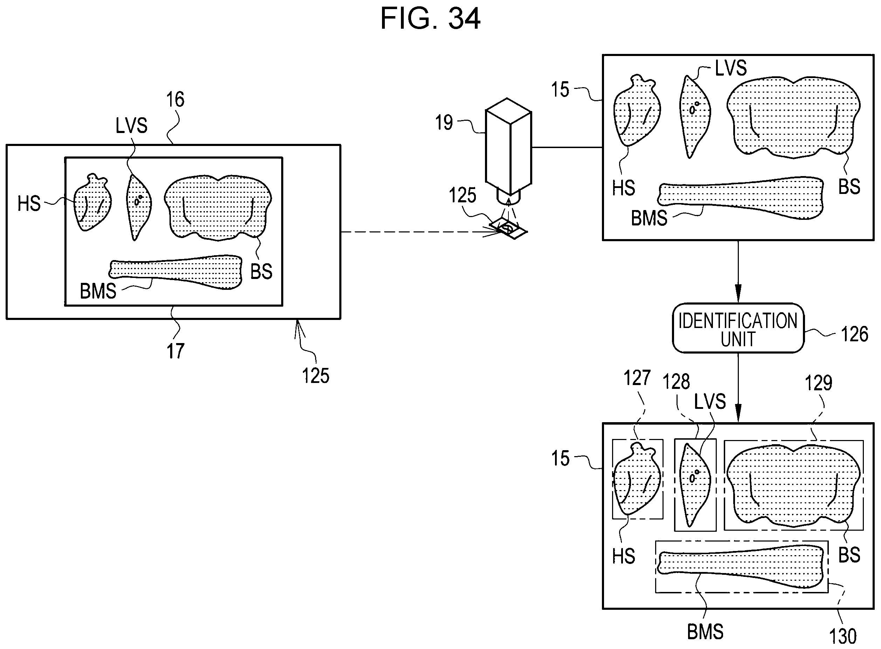

As illustrated in Fig. 34 as an example, in a fourth embodiment, a specimen slide 125 is handled in which tissue specimens of multiple types of organs are placed on a single glass slide 16. Fig. 34 illustrates a case where a liver specimen LVS, a heart specimen HS, a brain specimen BS, and a bone marrow specimen BMS are placed. In this case, the specimen image 15 includes the liver specimen LVS, the heart specimen HS, the brain specimen BS, and the bone marrow specimen BMS.

A CPU of a drug discovery support device according to the fourth embodiment functions as an identification unit 126 in addition to the processing units 50 to 55 according to the above-described first embodiment. The identification unit 126 identifies the tissue specimens of the respective organs from the specimen image 15 by using, for example, a template for identifying the tissue specimens of the respective organs or a machine learning model. The identification unit 126 outputs, as an identification result, coordinate information of frames 127 to 130 surrounding the tissue specimens of the respective organs. The frame 127 is a frame surrounding the heart specimen HS, and the frame 128 is a frame surrounding the liver specimen LVS. The frame 129 is a frame surrounding the brain specimen BS, and the frame 130 is a frame surrounding the bone marrow specimen BMS.

The detection unit 51 extracts the feature quantities 72 of the tissue specimens in the respective frames 127 to 130 by using dedicated feature quantity extractors 41. That is, the detection unit 51 extracts the feature quantities 72 of the tissue specimens of the respective organs identified by the identification unit 126. The subsequent process is the same as the process described in the first embodiment and so forth, and thus the illustration and description thereof will be omitted.

As described above, in the fourth embodiment, the specimen image 15 is an image obtained by imaging the specimen slide 125 on which tissue specimens of multiple types of organs are placed. The identification unit 126 identifies the tissue specimens of the respective organs from the specimen image 15. The detection unit 51 extracts the feature quantities 72 of each of the identified tissue specimens of the respective organs. Thus, it is possible to cope with the specimen slide 125 on which tissue specimens of multiple types of organs are placed. The specimen slide 125 on which tissue specimens of multiple types of organs are placed as in the present embodiment is more typical than the specimen slide 18 on which a tissue specimen of a single organ is placed as in the above-described first embodiment. Thus, it is possible to perform a process in accordance with a more typical operation. The frames 127 to 130 indicating the tissue specimens of the respective organs in the specimen image 15 may be defined by an operation of the user U.

The feature quantity 72 is not limited to the one extracted by the feature quantity extractor 41. The feature quantity 72 may be an average value, a maximum value, a minimum value, a mode value, a variance, or the like of the pixel values of the patch image 70.

The subject S is not limited to a rat. The subject S may be a mouse, a guinea pig, a gerbil, a hamster, a ferret, a rabbit, a dog, a cat, a monkey, or the like.

The drug discovery support device 10 may be a personal computer installed in a drug development facility as illustrated in Fig. 1, or may be a server computer installed in a data center independent of the drug development facility.

When the drug discovery support device 10 is constituted by a server computer, the specimen images 15 are transmitted from personal computers installed in individual drug development facilities to the server computer via a network such as the Internet. The server computer distributes various screens including the analysis instruction screen 90 to the personal computers in a format of screen data for web delivery created in a markup language such as an Extensible Markup Language (XML). Each personal computer reproduces, based on the screen data, a screen to be displayed on a web browser, and displays the reproduced screen on a display. Instead of the XML, another data description language such as JavaScript (registered trademark) Object Notation (JSON) may be used.

The drug discovery support device 10 according to the technology of the present disclosure can be widely used throughout all stages of drug development, from the setting of a drug discovery target in the earliest stage to a clinical trial in the final stage.

The candidate substance 27 is not limited to the exemplified drug. The candidate substance 27 may be another chemical substance such as an agricultural chemical or a radioactive substance.

The hardware configuration of the computer constituting the drug discovery support device 10 according to the technology of the present disclosure can be modified in various ways. For example, the drug discovery support device 10 may be constituted by multiple computers separated as hardware for the purpose of improving the processing capability and reliability. For example, the functions of the detection unit 51 and the determination unit 52 and the function of the information update unit 53 may be implemented by two computers in a distributed manner. In this case, the two computers constitute the drug discovery support device 10.

As described above, the hardware configuration of the computer of the drug discovery support device 10 can be changed as appropriate in accordance with a required performance such as processing capability, safety, and reliability. Furthermore, not only the hardware but also the application program such as the operation program 40 can be duplicated or stored in multiple storages in a distributed manner for the purpose of ensuring safety and reliability.

In each of the above-described embodiments, for example, the following various types of processors may be used as the hardware structures of the processing units that execute various processes, such as the RW control unit 50, the detection unit 51, the determination unit 52, the information update unit 53, the display control unit 54, the selection unit 55, the instruction receiving unit 114, and the identification unit 126. The various types of processors include, as described above, the CPU 32, which is a general-purpose processor that executes software (the operation program 40) and functions as various processing units; a programmable logic device (PLD), which is a processor whose circuit configuration is changeable after manufacturing, such as a field programmable gate array (FPGA); a dedicated electric circuit, which is a processor having a circuit configuration designed specifically for performing specific processing, such as an application specific integrated circuit (ASIC); and the like.

A single processing unit may be constituted by one of these various types of processors or may be constituted by a combination of two or more processors of the same type or different types (for example, a combination of multiple FPGAs, and/or a combination of a CPU and an FPGA). Multiple processing units may be constituted by a single processor.

Examples of constituting multiple processing units by a single processor are as follows. First, as represented by a computer of a client or server, a single processor is constituted by a combination of one or more CPUs and software, and the processor functions as multiple processing units. Secondly, as represented by a system on chip (SoC), a processor in which a single integrated circuit (IC) chip implements the function of an entire system including a plurality of processing units is used. In this way, various types of processing units are constituted by using one or more of the above-described various types of processors as a hardware structure.

Furthermore, as the hardware structure of the various types of processors, more specifically, electric circuitry formed by combining circuit elements such as semiconductor elements may be used.

From the above description, the technology described in the following appendices can be grasped.

In the technology of the present disclosure, the above-described various embodiments and/or various modifications can be combined as appropriate. The technology of the present disclosure is not limited to the above-described embodiments, and various configurations can be employed. The technology of the present disclosure includes, in addition to a program, a storage medium storing the program in a non-transitory manner.

The description given above and the illustration in the drawings are detailed description of the part related to the technology of the present disclosure and are merely an example of the technology of the present disclosure. For example, the description about the configurations, functions, operations, and effects given above is the description about an example of the configurations, functions, operations, and effects of the part related to the technology of the present disclosure. Thus, it goes without saying that an unnecessary part may be deleted from, a new element may be added to, or replacement may be performed on the description given above and the illustration in the drawings . To avoid complexity and facilitate understanding of the part related to the technology of the present disclosure, description of common technical knowledge or the like that is not particularly necessary to implement the technology of the present disclosure is omitted in the description given above and the illustration in the drawings.

In this specification, "A and/or B" is synonymous with "at least one of A or B". That is, "A and/or B" means only A, only B, or a combination of A and B. In this specification, a concept similar to "A and/or B" is applied to three or more things connected by "and/or".

## Claims

1. A drug discovery support device comprising a processor,
the processor being configured to:
obtain multiple specimen images of a tissue specimen of multiple organs of subjects subjected to an evaluation test of a candidate substance;
select, in accordance with degree-of-selection-priority information in which a degree of selection priority is set for each of the multiple organs, a target specimen image of a tissue specimen of a single organ from among the multiple specimen images;
make a determination as to whether a morphological abnormality has occurred in the tissue specimen in the target specimen image; and
update the degree-of-selection-priority information, based on a determination result as to whether the morphological abnormality has occurred.

2. The drug discovery support device according to claim 1, wherein in the degree-of-selection-priority information, a probability of being selected for the target specimen image is set as the degree of selection priority for each of the multiple organs.

3. The drug discovery support device according to claim 1 or 2, wherein the processor is configured to:
when the determination result indicates that the morphological abnormality has occurred, reset the degree of selection priority of the organ of the tissue specimen in the target specimen image to be higher; and
when the determination result indicates that the morphological abnormality has not occurred, keep the degree of selection priority of the organ of the tissue specimen in the target specimen image unchanged or reset the degree of selection priority of the organ of the tissue specimen in the target specimen image to be lower.

4. The drug discovery support device according to any one of claims 1 to 3, wherein the processor is configured to:
based on the determination result, reset the degree of selection priority of the organ of the tissue specimen in the target specimen image and also reset the degree of selection priority of a relevant organ having a functional relationship with the organ of the tissue specimen in the target specimen image.

5. The drug discovery support device according to any one of claims 1 to 4, wherein the processor is configured to:
receive from a user an input of a final determination result as to whether the morphological abnormality has actually occurred; and
update the degree-of-selection-priority information, based on the final determination result in addition to the determination result.

6. The drug discovery support device according to claim 5, wherein the processor is configured to:
when the determination result indicates that the morphological abnormality has not occurred but the final determination result indicates that the morphological abnormality has occurred, reset the degree of selection priority of the organ of the tissue specimen in the target specimen image to be higher; and
when the determination result indicates that the morphological abnormality has occurred but the final determination result indicates that the morphological abnormality has not occurred, keep the degree of selection priority of the organ of the tissue specimen in the target specimen image unchanged or reset the degree of selection priority of the organ of the tissue specimen in the target specimen image to be lower.

7. The drug discovery support device according to any one of claims 1 to 6, wherein
the subjects are grouped into multiple groups, and
in the degree-of-selection-priority information, the degree of selection priority is set for each of the multiple organs and for each of the multiple groups.

8. The drug discovery support device according to claim 7, wherein the multiple groups include an administration group to which the candidate substance is administered and a control group to which the candidate substance is not administered.

9. The drug discovery support device according to claim 8, wherein the administration group includes multiple sub-administration groups that are different from each other in a dose of the candidate substance.

10. The drug discovery support device according to any one of claims 1 to 9, wherein in the degree-of-selection-priority information in an initial state, a degree of selection priority based on knowledge that has already been obtained is set.

11. The drug discovery support device according to any one of claims 1 to 10, wherein the processor is configured to:
detect, from among portions of the target specimen image, one or more estimated morphological abnormality portions where the morphological abnormality is estimated to have occurred; and
compare a numerical value related to the number of the one or more estimated morphological abnormality portions with a determination threshold value set in advance, and thus make the determination.

12. The drug discovery support device according to claim 11, wherein the processor is configured to:
handle each of multiple patch images obtained by dividing the target specimen image as one of the portions; and
compare feature quantities obtained by inputting the patch images to a machine learning model with reference feature quantities obtained by inputting reference patch images of a tissue specimen regarded to be normal to the machine learning model, and thus detect the one or more estimated morphological abnormality portions.

13. A method for operating a drug discovery support device, the method comprising:
obtaining multiple specimen images of a tissue specimen of multiple organs of subjects subjected to an evaluation test of a candidate substance;
selecting, in accordance with degree-of-selection-priority information in which a degree of selection priority is set for each of the multiple organs, a target specimen image of a tissue specimen of a single organ from among the multiple specimen images;
making a determination as to whether a morphological abnormality has occurred in the tissue specimen in the target specimen image; and
updating the degree-of-selection-priority information, based on a determination result as to whether the morphological abnormality has occurred.

14. A computer-readable storage medium storing a program for operating a drug discovery support device, the program causing a computer to execute a process comprising:
obtaining multiple specimen images of a tissue specimen of multiple organs of subjects subjected to an evaluation test of a candidate substance;
selecting, in accordance with degree-of-selection-priority information in which a degree of selection priority is set for each of the multiple organs, a target specimen image of a tissue specimen of a single organ from among the multiple specimen images;
making a determination as to whether a morphological abnormality has occurred in the tissue specimen in the target specimen image; and
updating the degree-of-selection-priority information, based on a determination result as to whether the morphological abnormality has occurred.

## Patentansprüche

1. Unterstützungsvorrichtung für Arzneimittelforschung, umfassend einen Prozessor,
wobei der Prozessor so konfiguriert ist, dass er:
mehrere Probenbilder einer Gewebeprobe von mehreren Organen von Untersuchungspersonen, die einem Bewertungstest einer Kandidatensubstanz unterzogen worden sind, erhält;
gemäß Informationen über Auswahlprioritätsgrad, in denen ein Auswahlprioritätsgrad für jedes der mehreren Organe eingestellt ist, ein Zielprobenbild einer Gewebeprobe eines einzelnen Organs aus den mehreren Probenbildern auswählt;
eine Bestimmung darüber, ob eine morphologische Abnormalität in der Gewebeprobe in dem Zielprobenbild aufgetreten ist, vornimmt; und
die Informationen über Auswahlprioritätsgrad auf der Grundlage eines Bestimmungsergebnisses darüber, ob die morphologische Abnormalität aufgetreten ist, aktualisiert.

2. Unterstützungsvorrichtung für Arzneimittelforschung nach Anspruch 1, wobei in den Informationen über Auswahlprioritätsgrad eine Wahrscheinlichkeit, für das Zielprobenbild ausgewählt zu werden, als der Auswahlprioritätsgrad für jedes der mehreren Organe eingestellt ist.

3. Unterstützungsvorrichtung für Arzneimittelforschung nach Anspruch 1 oder 2, wobei der Prozessor so konfiguriert ist, dass er:
wenn das Bestimmungsergebnis angibt, dass die morphologische Abnormalität aufgetreten ist, den Auswahlprioritätsgrad des Organs der Gewebeprobe in dem Zielprobenbild so zurücksetzt, dass er höher ist; und
wenn das Bestimmungsergebnis angibt, dass die morphologische Abnormalität nicht aufgetreten ist, den Auswahlprioritätsgrad des Organs der Gewebeprobe in dem Zielprobenbild unverändert hält oder den Auswahlprioritätsgrad des Organs der Gewebeprobe in dem Zielprobenbild so zurücksetzt, dass er niedriger ist.

4. Unterstützungsvorrichtung für Arzneimittelforschung nach einem der Ansprüche 1 bis 3, wobei der Prozessor so konfiguriert ist, dass er:
auf der Grundlage des Bestimmungsergebnisses den Auswahlprioritätsgrad des Organs der Gewebeprobe in dem Zielprobenbild zurücksetzt und auch den Auswahlprioritätsgrad eines relevanten Organs, das eine funktionale Beziehung zu dem Organ der Gewebeprobe in dem Zielprobenbild aufweist, zurücksetzt.

5. Unterstützungsvorrichtung für Arzneimittelforschung nach einem der Ansprüche 1 bis 4, wobei der Prozessor so konfiguriert ist, dass er:
eine Eingabe eines endgültigen Bestimmungsergebnisses darüber, ob die morphologische Abnormalität tatsächlich aufgetreten ist, von einem Benutzer empfängt; und
die Informationen über Auswahlprioritätsgrad auf der Grundlage des endgültigen Bestimmungsergebnisses zusätzlich zu dem Bestimmungsergebnis aktualisiert.

6. Unterstützungsvorrichtung für Arzneimittelforschung nach Anspruch 5, wobei der Prozessor so konfiguriert ist, dass er:
wenn das Bestimmungsergebnis angibt, dass die morphologische Abnormalität nicht aufgetreten ist, aber das endgültige Bestimmungsergebnis angibt, dass die morphologische Abnormalität aufgetreten ist, den Auswahlprioritätsgrad des Organs der Gewebeprobe in dem Zielprobenbild so zurücksetzt, dass er höher ist; und
wenn das Bestimmungsergebnis angibt, dass die morphologische Abnormalität aufgetreten ist, aber das endgültige Bestimmungsergebnis angibt, dass die morphologische Abnormalität nicht aufgetreten ist, den Auswahlprioritätsgrad des Organs der Gewebeprobe in dem Zielprobenbild unverändert hält oder den Auswahlprioritätsgrad des Organs der Gewebeprobe in dem Zielprobenbild so zurücksetzt, dass er niedriger ist.

7. Unterstützungsvorrichtung für Arzneimittelforschung nach einem der Ansprüche 1 bis 6, wobei die Untersuchungspersonen in mehrere Gruppen gruppiert sind, und
in den Informationen über Auswahlprioritätsgrad der Auswahlprioritätsgrad für jedes der mehreren Organe und für jede der mehreren Gruppen eingestellt ist.

8. Unterstützungsvorrichtung für Arzneimittelforschung nach Anspruch 7, wobei die mehreren Gruppen eine Verabreichungsgruppe, der die Kandidatensubstanz verabreicht wird, und eine Kontrollgruppe, der die Kandidatensubstanz nicht verabreicht wird, enthalten.

9. Unterstützungsvorrichtung für Arzneimittelforschung nach Anspruch 8, wobei die Verabreichungsgruppe mehrere Unter-Verabreichungsgruppen enthält, die sich in einer Dosis der Kandidatensubstanz voneinander unterscheiden.

10. Unterstützungsvorrichtung für Arzneimittelforschung nach einem der Ansprüche 1 bis 9, wobei in den Informationen über Auswahlprioritätsgrad in einem Anfangszustand ein Auswahlprioritätsgrad auf der Grundlage von bereits erhaltenen Kenntnissen eingestellt ist.

11. Unterstützungsvorrichtung für Arzneimittelforschung nach einem der Ansprüche 1 bis 10, wobei der Prozessor so konfiguriert ist, dass er:
einen oder mehrere geschätzte morphologische Abnormalitätsabschnitte, in denen geschätzt wird, dass die morphologische Abnormalität aufgetreten ist, aus den Abschnitten des Zielprobenbildes detektiert; und
einen numerischen Wert, der sich auf die Anzahl der einen oder mehreren geschätzten morphologischen Abnormalitätsabschnitte bezieht, mit einem im Voraus eingestellten Bestimmungsschwellenwert vergleicht und somit die Bestimmung vornimmt.

12. Unterstützungsvorrichtung für Arzneimittelforschung nach Anspruch 11, wobei der Prozessor so konfiguriert ist, dass er:
jedes von mehreren Patch-Bildern, die durch Unterteilen des Zielprobenbildes erhalten werden, als einen der Abschnitte behandelt; und
Merkmalsmengen, die durch Eingeben der Patch-Bilder in ein Modell für maschinelles Lernen erhalten werden, mit Referenzmerkmalsmengen, die durch Eingeben von Referenz-Patch-Bildern einer Gewebeprobe, die als normal angesehen wird, in das Modell für maschinelles Lernen erhalten werden, vergleicht und somit den einen oder die mehreren geschätzten morphologischen Abnormalitätsabschnitte detektiert.

13. Verfahren zum Betreiben einer Unterstützungsvorrichtung für Arzneimittelforschung, wobei das Verfahren umfasst:
Erhalten mehrerer Probenbilder einer Gewebeprobe von mehreren Organen von Untersuchungspersonen, die einem Bewertungstest einer Kandidatensubstanz unterzogen worden sind;
Auswählen, in Übereinstimmung mit Informationen über Auswahlprioritätsgrad, in denen ein Auswahlprioritätsgrad für jedes der mehreren Organe eingestellt ist, eines Zielprobenbildes einer Gewebeprobe eines einzelnen Organs aus den mehreren Probenbildern;
Vornehmen einer Bestimmung darüber, ob eine morphologische Abnormalität in der Gewebeprobe in dem Zielprobenbild aufgetreten ist; und
Aktualisieren der Informationen über Auswahlprioritätsgrad auf der Grundlage eines Bestimmungsergebnisses darüber, ob die morphologische Abnormalität aufgetreten ist.

14. Computerlesbares Speichermedium, das ein Programm zum Betreiben einer Unterstützungsvorrichtung für Arzneimittelforschung speichert, wobei das Programm einen Computer veranlasst, einen Prozess auszuführen, der umfasst:
Erhalten mehrerer Probenbilder einer Gewebeprobe von mehreren Organen von Untersuchungspersonen, die einem Bewertungstest einer Kandidatensubstanz unterzogen worden sind;
Auswählen, in Übereinstimmung mit Informationen über Auswahlprioritätsgrad, in denen ein Auswahlprioritätsgrad für jedes der mehreren Organe eingestellt ist, eines Zielprobenbildes einer Gewebeprobe eines einzelnen Organs aus den mehreren Probenbildern;
Vornehmen einer Bestimmung darüber, ob eine morphologische Abnormalität in der Gewebeprobe in dem Zielprobenbild aufgetreten ist; und
Aktualisieren der Informationen über Auswahlprioritätsgrad auf der Grundlage eines Bestimmungsergebnisses darüber, ob die morphologische Abnormalität aufgetreten ist.

## Revendications

1. Dispositif d'aide à la découverte de médicaments comprenant un processeur,
le processeur étant configuré pour :
obtenir plusieurs images d'échantillon d'un échantillon de tissu provenant de plusieurs organes de sujets soumis à un test d'évaluation d'une substance candidate ;
sélectionner, conformément à des informations de degré de priorité de sélection dans lesquelles un degré de priorité de sélection est défini pour chacun des plusieurs organes, une image d'échantillon cible d'un échantillon de tissu provenant d'un seul organe parmi les plusieurs images d'échantillon ;
effectuer une détermination quant à savoir si une anomalie morphologique s'est produite dans l'échantillon de tissu dans l'image d'échantillon cible ; et
mettre à jour les informations de degré de priorité de sélection, sur la base d'un résultat de détermination indiquant si l'anomalie morphologique s'est produite.

2. Dispositif d'aide à la découverte de médicaments selon la revendication 1, dans lequel dans les informations de degré de priorité de sélection, une probabilité d'être sélectionné pour l'image d'échantillon cible est définie comme degré de priorité de sélection pour chacun des plusieurs organes.

3. Dispositif d'aide à la découverte de médicaments selon la revendication 1 ou la revendication 2, dans lequel le processeur est configuré pour :
lorsque le résultat de détermination indique que l'anomalie morphologique s'est produite, redéfinir à une valeur plus élevée le degré de priorité de sélection de l'organe de l'échantillon de tissu dans l'image d'échantillon cible ; et
lorsque le résultat de détermination indique que l'anomalie morphologique ne s'est pas produite, maintenir inchangé le degré de priorité de sélection de l'organe de l'échantillon de tissu dans l'image d'échantillon cible ou redéfinir à une valeur plus faible le degré de priorité de sélection de l'organe de l'échantillon de tissu dans l'image d'échantillon cible.

4. Dispositif d'aide à la découverte de médicaments selon l'une quelconque des revendications 1 à 3, dans lequel le processeur est configuré pour :
sur la base du résultat de détermination, redéfinir le degré de priorité de sélection de l'organe de l'échantillon de tissu dans l'image d'échantillon cible et redéfinir également le degré de priorité de sélection d'un organe pertinent ayant une relation fonctionnelle avec l'organe de l'échantillon de tissu dans l'image d'échantillon cible.

5. Dispositif d'aide à la découverte de médicaments selon l'une quelconque des revendications 1 à 4, dans lequel le processeur est configuré pour :
recevoir d'un utilisateur une entrée d'un résultat de détermination final indiquant si l'anomalie morphologique s'est effectivement produite ; et
mettre à jour les informations de degré de priorité de sélection, sur la base du résultat de détermination final en plus du résultat de détermination.

6. Dispositif d'aide à la découverte de médicaments selon la revendication 5, dans lequel le processeur est configuré pour :
lorsque le résultat de détermination indique que l'anomalie morphologique ne s'est pas produite, mais que le résultat de détermination final indique que l'anomalie morphologique s'est produite, redéfinir à une valeur plus élevée le degré de priorité de sélection de l'organe de l'échantillon de tissu dans l'image d'échantillon cible ; et
lorsque le résultat de détermination indique que l'anomalie morphologique s'est produite, mais que le résultat de détermination final indique que l'anomalie morphologique ne s'est pas produite, maintenir inchangé le degré de priorité de sélection de l'organe de l'échantillon de tissu dans l'image d'échantillon cible ou redéfinir à une valeur plus faible le degré de priorité de sélection de l'organe de l'échantillon de tissu dans l'image d'échantillon cible.

7. Dispositif d'aide à la découverte de médicaments selon l'une quelconque des revendications 1 à 6, dans lequel les sujets sont regroupés en plusieurs groupes, et dans les informations de degré de priorité de sélection, le degré de priorité de sélection est défini pour chacun des plusieurs organes et pour chacun des plusieurs groupes.

8. Dispositif d'aide à la découverte de médicaments selon la revendication 7, dans lequel les plusieurs groupes incluent un groupe d'administration auquel la substance candidate est administrée et un groupe témoin auquel la substance candidate n'est pas administrée.

9. Dispositif d'aide à la découverte de médicaments selon la revendication 8, dans lequel le groupe d'administration inclut plusieurs sous-groupes d'administration qui sont différents les uns des autres par une dose de la substance candidate.

10. Dispositif d'aide à la découverte de médicaments selon l'une quelconque des revendications 1 à 9, dans lequel dans les informations de degré de priorité de sélection dans un état initial, un degré de priorité de sélection fondé sur des connaissances déjà obtenues est défini.

11. Dispositif d'aide à la découverte de médicaments selon l'une quelconque des revendications 1 à 10, dans lequel le processeur est configuré pour :
détecter, parmi des portions de l'image d'échantillon cible, une ou plusieurs portions estimées comme présentant une anomalie morphologique où l'on estime que l'anomalie morphologique s'est produite ; et
comparer une valeur numérique liée au nombre de l'une ou plusieurs portions estimées comme présentant une anomalie morphologique avec une valeur seuil de détermination définie à l'avance, et ainsi effectuer la détermination.

12. Dispositif d'aide à la découverte de médicaments selon la revendication 11, dans lequel le processeur est configuré pour :
traiter chacune de plusieurs images de patch obtenues en divisant l'image d'échantillon cible comme l'une des portions ; et
comparer des quantités de caractéristiques obtenues en entrant les images de patch dans un modèle d'apprentissage automatique avec des quantités de caractéristiques de référence obtenues en entrant des images de patch de référence d'un échantillon de tissu considéré comme normal dans le modèle d'apprentissage automatique, et ainsi détecter l'une ou plusieurs portions estimées comme présentant une anomalie morphologique.

13. Procédé de fonctionnement d'un dispositif d'aide à la découverte de médicaments, le procédé comprenant :
obtenir plusieurs images d'échantillon d'un échantillon de tissu provenant de plusieurs organes de sujets soumis à un test d'évaluation d'une substance candidate ;
sélectionner, conformément à des informations de degré de priorité de sélection dans lesquelles un degré de priorité de sélection est défini pour chacun des plusieurs organes, une image d'échantillon cible d'un échantillon de tissu provenant d'un seul organe parmi les plusieurs images d'échantillon ;
effectuer une détermination quant à savoir si une anomalie morphologique s'est produite dans l'échantillon de tissu dans l'image d'échantillon cible ; et
mettre à jour les informations de degré de priorité de sélection, sur la base d'un résultat de détermination indiquant si l'anomalie morphologique s'est produite.

14. Support de stockage lisible par ordinateur stockant un programme pour faire fonctionner un dispositif d'aide à la découverte de médicaments, le programme amenant un ordinateur à exécuter un processus comprenant :
obtenir plusieurs images d'échantillon d'un échantillon de tissu provenant de plusieurs organes de sujets soumis à un test d'évaluation d'une substance candidate ;
sélectionner, conformément à des informations de degré de priorité de sélection dans lesquelles un degré de priorité de sélection est défini pour chacun des plusieurs organes, une image d'échantillon cible d'un échantillon de tissu provenant d'un seul organe parmi les plusieurs images d'échantillon ;
effectuer une détermination quant à savoir si une anomalie morphologique s'est produite dans l'échantillon de tissu dans l'image d'échantillon cible ; et
mettre à jour les informations de degré de priorité de sélection, sur la base d'un résultat de détermination indiquant si l'anomalie morphologique s'est produite.
